(19) Europäisches Patentamt
European Patent Office
Office européen des brevets

(11) Numéro de publication: **0 174 242 B1**

(12) # FASCICULE DE BREVET EUROPÉEN

(45) Date de publication du fascicule du brevet: **13.01.88**

(21) Numéro de dépôt: **85401624.3**

(22) Date de dépôt: **09.08.85**

(51) Int. Cl.⁴: **C 07 C 97/10**, C 07 D 295/10, A 61 K 31/00 // C07C103/42, C07C103/44

(54) **Dérivés de 1-(aminophényl)-2-amino-propanone, leur utilisation en thérapeutique et leur procédé de préparation.**

(30) Priorité: **20.08.84 FR 8412962**

(43) Date de publication de la demande: **12.03.86 Bulletin 86/11**

(45) Mention de la délivrance du brevet: **13.01.88 Bulletin 88/2**

(84) Etats contractants désignés: **AT BE CH DE FR GB IT LI LU NL SE**

(56) Documents cité:
**EP-A-0 138 714**
**GB-A-1 180 890**

**CHEMICAL ABSTRACTS, vol. 40, no. 7, 10 avril 1946, colonne 2129-2130-8, Columbus, Ohio, US; W.H. HARTUNG et al.: "Amino alcohols. XV. Rho-aminopropadrine"**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 174 242 B1

**Description**

La présente invention a trait à de nouveaux dérivés de 1-(aminophényl)-2-amino-propanone. Elle concerne également l'utilisation de ces nouveaux dérivés en thérapeutique, notamment en tant qu'agents antidépresseurs du système nerveux central (SNC), vasodilatateurs et/ou immunostimulants, d'une part, et leur procédé de préparation, d'autre part.

On sait que l'on a déjà décrit des dérivés de (aminophényl)amino-alcanone. On connaît en particulier de FR-A-2 035 155 les 1-(4-aminophényl)-4-morpholino-butanone, 1-(4-aminophényl)-4-(3-méthyl-morpholinyl)-butanone et 1-(4-aminophényl)-4-(3-tertiobutyl-morpholinyl)-butanone en tant qu'agents inhibiteurs sélectifs de la mono-amine oxydase. On connaît également de GB-A-1 180 890 le chlorhydrate de 1-(4-amino-3-chlorophényl)-2-(3-méthoxy-propylamino)-propanone (cf exemple 44) et la 1-(4-amino-3-bromophényl)-2-(2-éthylpipéridino)-propanone (cf exemple 45) en tant qu'intermédiaires de synthèse, les propanols correspondants ayant des propriétés analgésiques.

On vient de trouver que les dérivés de 1-(aminophényl)-2-amino-propanone selon l'invention, qui sont structurellement différents des dérivés précités, ont des propriétés thérapeutiques intéressantes. Les nouveaux dérivés selon l'invention agissent tous sur le SNC, ils ont en particulier des effets antidépresseurs. A côté de ces effets antidépresseurs communs à l'ensemble, on constate que ces dérivés peuvent présenter des effets psychostimulants, immunologiques et/ou cardiovasculaires qui sont bénéfiques comme indiqué ci-après.

Les nouveaux dérivés selon l'invention sont caractérisés en ce qu ils sont choisis parmi l'ensemble constitué par

(a) les 1-(aminophényl)-2-amino-propanones répondant à la formule générale

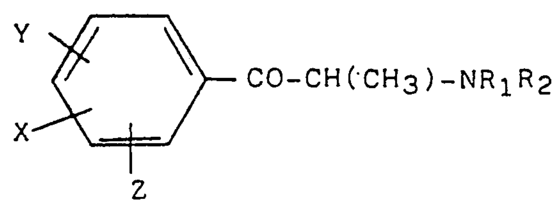
(I)

dans laquelle

X est $NH_2$,

Y est un atome d'hydrogène ou d'halogène,

Z est un atome d'hydrogène ou d'halogène,

$R_1$ est un groupe alkyle en $C_1$-$C_4$ ou un groupe cycloalkyle en $C_3$-$C_6$,

$R_2$ est l'atome d'hydrogène ou un groupe alkyle en $C_1$-$C_4$,

$R_1$ et $R_2$, considérés ensemble, peuvent former avec l'atome d'azote auquel ils sont liés à un groupe N-hétérocyclique de 5 à 7 sommets, pouvant comporter un second hétéroatome choisi parmi N, O et S et pouvant être substitué, ledit groupe hétérocyclique $NR_1R_2$ étant choisi parmi l'ensemble comprenant les groupes pyrrolidino, morpholino, thiomorpholino, pipéridino, hexaméthylèneimino, pipérazino, 4-méthyl-pipérazino, 4-(β-hydroxyéthyl)-pipérazino, 4-phénylpipérazino, 4-(p-chloro-phényl)-pipérazino; et,

b) leurs sels d'addition.

Parmi les groupes alkyle en $C_1$-$C_4$ inclus dans les définitions des groupes $R_1$ et $R_2$ on peut notamment citer les groupes $CH_3$, $CH_2CH_3$, $CH_2CH_2CH_3$, $CH(CH_3)_2$, $C(CH_3)_3$, $CH(CH_3)CH_2CH_3$, $CH_2CH(CH_3)_2$ et $CH_2CH_2CH_2CH_3$.

Parmi les groupes cycloalkyle en $C_3$-$C_6$ inclus dans la définition du groupe $R_1$, on peut mentionner notamment les groupes cyclopropyle, cyclopentyle et cyclohexyle.

Les groupes N-hétérocycliques $NR_1R_2$ qui conviennent selon l'invention sont avantageusement saturés. Ils comprennent de 5 à 7 sommets, peuvent comporter un second hétéroatome choisi parmi l'ensemble constitué par N, O et S, et, peuvent être substitués comme indiqué ci-dessus.

Parmi les atomes d'halogène inclus dans les définitions des groupes Y et Z on peut notamment citer F, Cl et Br qui conviennent, l'atome d'halogène préféré étant ici Cl.

Sur le groupe phényle le groupe amino ($X=NH_2$) peut, compte-tenu des définitions données ci-dessus, être en position ortho, méta et para par rapport au groupe carbonyle. De préférence ce groupe amino sera situé en position para ou méta.

Les groupes $XYZC_6H_2$ préférés sont les groupes 4-aminophényle, 4-amino-3-chlorophényle, 4-amino-3,5-dichlorophényle, 3-aminophényle et 3-amino-4-chlorophényle.

Par sels d'addition, on entend ici les sels d'addition d'acide obtenus par réaction d'une des bases libres susvisées avec un acide minéral ou organique, d'une part, et les sels d'ammonium, d'autre part. Parmi les acides utilisables pour salifier les bases libres susvisées, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Un certain nombre de composés selon l'invention à été consigné de façon nullement limitative dans le tableu

I ci-après. Les points de fusion qui ont été donnés sont des points de fusion instantanée déterminés au banc Kofler.

**TABLEAU I**

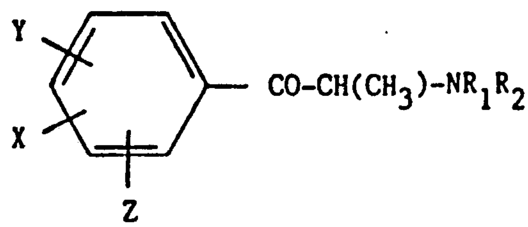

| PRODUIT | N° DE CODE | X | Y | Z | $NR_1R_2$ | F(°C) |
|---|---|---|---|---|---|---|
| Ex 1 (a) | CRl 41 153 | $4\text{-}NH_2$ | H | H | $NHCH(CH_3)_2$ | 210 |
| Ex 2 (a) | CRL 41 178 | $4\text{-}NH_2$ | H | H | $NHC(CH_3)_3$ | 250 (d) |
| Ex 3 (b) | CRL 41 194 | $4\text{-}NH_2$ | 3-Cl | 5-Cl | $NHCH(CH_3)_2$ | (e) |
| Ex 4 (a) | CRL 41 213 | $4\text{-}NH_2$ | H | H | $NHCH_2CH_2CH_3$ | 160 (d) |
| Ex 5 (a) | CRL 41 218 | $4\text{-}NH_2$ | H | H | $NHCH_3$ | 220 (d) |
| Ex 6 (b) | CRL 41 221 | $4\text{-}NH_2$ | 3-Cl | H | $NHCH(CH_3)_2$ | (e) |
| Ex 7 (a) | CRL 41 222 | $4\text{-}NH_2$ | H | H | 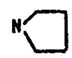 | 200 |
| Ex 8 (a) | CRL 41 225 | $4\text{-}NH_2$ | H | H | $NHCH_2CH_3$ | 190 (d) |
| Ex 9 (a) | CRL 41 233 | $4\text{-}NH_2$ | H | H | $N(CH_3)_2$ | 180 (d) |
| Ex 10 (a) | CRL 41 237 | $4\text{-}NH_2$ | H | H | 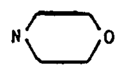 | 210 (d) |
| Ex 11 (a) | CRL 41 241 | $4\text{-}NH_2$ | H | H | 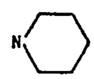 | 250 (d) |
| Ex 12 (c) | CRL 41 243 | $4\text{-}NH_2$ | H | H | 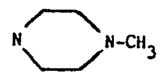 | 230 (d) |
| Ex 13 (a) | CRL 41 246 | $4\text{-}NH_2$ | H | H | 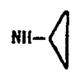 | 150 (d) |
| EX 14 (a) | CRL 41 248 | $4\text{-}NH_2$ | H | H | 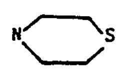 | 190-200 (d) |
| Ex 15 (a) | CRL 41 255 | $3\text{-}NH_2$ | H | H | $NHCH(CH_3)_2$ | 200 (d) |
| Ex 16 (a) | CRL 41 260 | $3\text{-}NH_2$ | H | H | $N(CH_3)_2$ | 210 (d) |
| Ex 17 (a) | CRL 41 263 | $3\text{-}NH_2$ | H | H | 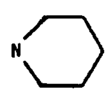 | 200 (d) |
| Ex 18 (a) | CRL 41 268 | $3\text{-}NH_2$ | H | H | 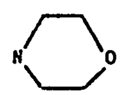 | 210 (d) |
| Ex 19 (a) | CRL 41 278 | $3\text{-}NH_2$ | H | H | $NHCH_2CH_2CH_3$ | 190 (d) |

<u>Notes</u>
a) : dichlorhydrate
b) : monochlorhydrate
c) : trichlorhydrate
d) : avec décomposition
e) : supérieur à 260°C.

Les composés préférés selon l'invention comprennent les 1-(4-aminophényl)-2-isopropylamino-, 1-(4-

aminophényl)-2-tertiobutylamino-, 1-(4-amino-3-chlorophényl)-2-isopropylamino-, 1-(4-aminophényl)-2-pyrrolidino-, 1-(4-aminophényl)-2-éthylamino-, 1-(4-aminophényl)-2-diméthylamino-, 1-(4-aminophényl)-2-cyclopropylamino-propanones et leurs sels d'addition, et, surtout les 1-(4-amino-3,5-dichlorophé-nyl)-2-isopropylamino- et 1-(4-aminophényl)-2-morpholino-propanones et leurs sels d'addition.

Les nouveaux dérivés de 1-(aminophényl)-2-amino-propanone peuvent être préparés selon une méthode connue en soi par application de mecanismes réactionnels classiques. On préconise selon l'invention, pour préparer ces dérivés, deux méthodes de synthèse schématisés par les réactions suivantes:

**Variante A**

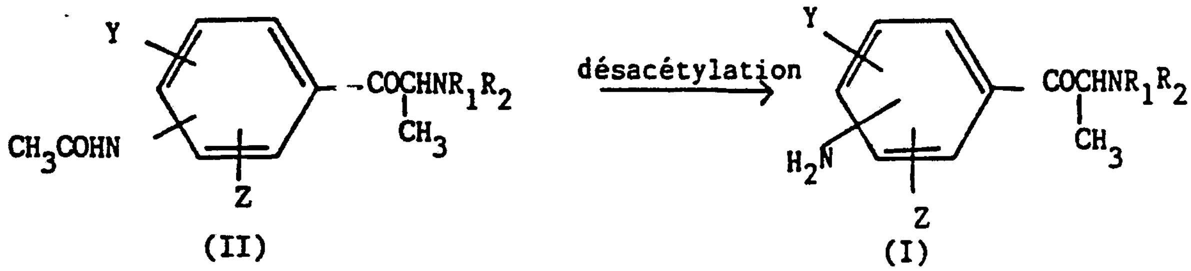


(II) (I)

**Variante B**

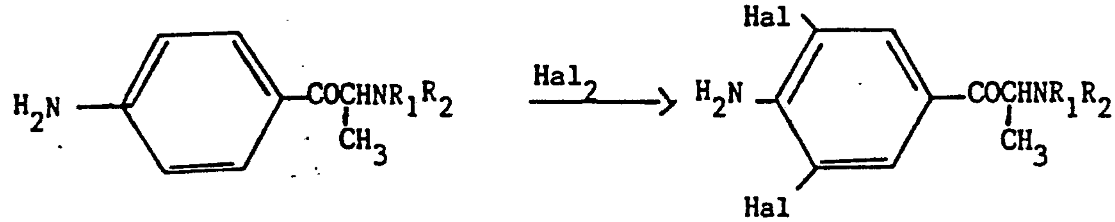


($I_a$) ($I_b$)

La variante A selon l'invention consiste à soumettre un dérivé de 1-(acétylaminophényl)-2-amino-propanone de formule II (où Y, Z, $R_1$ et $R_2$ sont définis comme indiqués ci-dessus) à une réaction de désacétylation au moyen de HCl en solution aqueuse pendant au moins 0,25 h à la température de reflux de milieu réactionnel.

De façon avantageuse la désacétylation est réalisée en faisant réagir 1 mole de composé II avec 0,7 l à 2 l d'acide HCl 4N pendant 0,25 h à 1 h à la température de reflux du milieu réactionnel.

La variante B a trait à un mode particulier de préparation de composés de formule I où $X=4-NH_2$ et $Y=Z=Hal$ (Hal désignant un atome d'halogène, notamment F, Cl et Br, et de préférence Cl).

Selon la variante B on soumet un composé de formule $I_a$ (où $R_1$ et $R_2$ sont définis comme indiqué ci-dessus) à une réaction d'halogénation en milieu aqueux au moyen d'un courant gazeux de $Hal_2$ pendant au moins 0,25 h. De façon avantageuse, pour la mise en oeuvre du procédé selon la variante B, on fait réagir dans de l'eau 2 moles de $Hal_2$ avec 1 mole de 1-(aminophényl)-2-amino-propanone de formule $I_a$, à une température comprise entre 8° C et 15° C (de préférence 10° C), pendant au moins 0,25 h (de préférence pendant 0,25 h à 1 h).

Bien entendu la variante A est la plus commode en ce sens qu'elle s'applique à la synthèse de l'ensemble des composés de formule I selon l'invention.

Les nouveaux dérivés de 1-(aminophényl)-2-amino-propanone selon l'invention sont utiles en thérapeutique. Ils agissent tous sur le SNC, en particulier ils présentent tous des effets antidépresseurs. Les résultats des essais neuropsychopharmacologiques montrent que la plupart de ces dérivés ont des effets stimulants ou excitants. Certains de ces composés présentent en outre, à côté des effets communs sur le SNC (tels que les effets antidépresseurs qui se manifestent avec une intensité plus ou moins importante), des propriétés immunologiques et/ou cardiovasculaires intéressantes. En particulier les produits des exemples 3 (CRL 41 194), 6 (CRL 41 221), 10 (CRL 41 237) et 14 (CRL 41 248) possèdent des effets immunostimulants ou immunomodulateurs.

Les produits les plus intéressants sur le plan pharmaceutique sont:

a) <u>en raison de leurs propriétés neuropsychopharmacologiques</u>: les produits des exemples 1 (CRL 41 153), 2 (CRL 41 178) et 4 (CRL 41 213);

b) <u>en raison de leurs propriétés vasodilatatrices</u>: les produits des exemples 6 (CRL 41 221), 7 (CRL 41 222), 8 (CRL 41 225), 9 (CRL 41 233) et 13 (CRL 41 246); et

c) <u>surtout</u> le produit de l'exemple 3 (CRL 41 194) qui est un bon agent vasodilatateur et est doué de

propriétés immunologiques bénéfiques, et le produit de l'exemple 10 (CRL 41 237) qui est un bon agent antidépresseur et est doué d'effets immunostimulants.

Selon l'invention on préconise une composition thérapeutique qui est caractérisée en ce qu'elle renferme, en association avec un excipient physiologiquement acceptable, au moins un dérivé de 1-(aminophényl)-2-amino-propanone ou l'un de ses sels d'addition non-toxiques, en tant que principe actif.

Bien entendu, dans une telle composition le principe actif intervient en quantité pharmaceutiquement efficace.

D'autres avantages et caractéristiques de l'invention seront mieux compris à la lecture qui va suivre, d'une part, d'exemples de préparation, et, d'autre part, de résultats d'essais pharmacologiques, l'ensemble de ces éléments n'étant nullement limitatif mais donné à titre d'illustration.

**PREPARATION I**

Obtention du chlorhydrate de 1-(4-amino-3-chlorophényl)-2-isopropylamino-propanone.

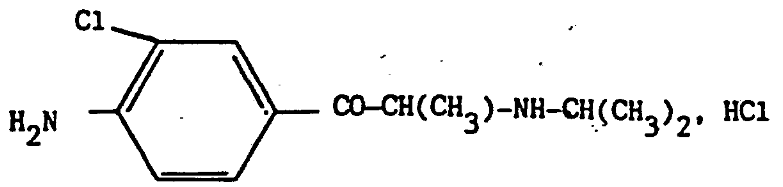

(Exemple 6; N° de Code: CRL 41 221)

a) 1-(4-Acétylamio-3-chlorophényl)-2-chloro-propanone

Dans 400 ml de chloroforme on met en suspension 45 g de 1-(4-acétylaminophényl)-2-chloro-propanone. On introduit par barbotage 0,2 mole de chlore. Le milieu réactionnel résultant (qui est une solution de couleur jaune) est évaporé à sec et par recristallisation du résidu d'évaporation dans le toluène on obtient 26 g (rendement: 50 %) de 1-(4-acétylamino-3-chlorophényl)-2-chloro-propanone. $F_{inst}=118°C$.

b) Chlorhydrate de 1-(4-acétylamino-3-chlorophényl)-2-isopropylamino-propanone.

On dissout 26 g de 1-(4-acetylamino-3-chlorophényl)-2-chloro-propanone dans 150 ml d'isopropylamine puis porte au reflux pendant 4 h le milieu réactionnel résultant. L'isopropylamine en excès est évaporée sous vide, le produit huileux restant est repris dans de l'éthanol et on précipite le chlorhydrate attendu au moyen de HCl gaz pour obtenir 9,6 g (rendement: 15 %) de chlorhydrate de 1-(4-acétylamino-3-chlorophényl)-2-isopropylamino-propanone. $F_{inst}=260°C$.

c) CRL 41 221

On dissout 9,6 g de chlorhydrate de 1-(4-acétylamino-3-chlorophényl)-2-isopropylamino-propanone dans 50 ml HCl 4N, puis porte au reflux pendant 0,5 h. Après évaporation sous vide et recristallisation dans l'éthanol, on obtient 7,6 g (rendement: 13,7 %) de CRL 41 221. $F_{inst}=260°C$.

**PREPARATION II**

Obtention du dichlorhydrate de 1-(4-aminophényl)-2-pyrrolidino-propanone.

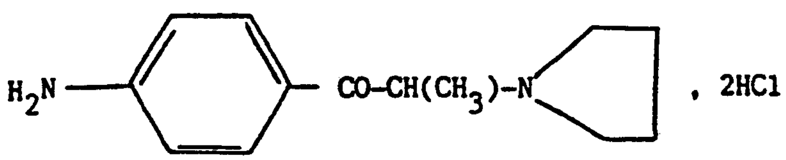

(Exemple 7; N° de Code : CRL 41 222).

On dissout 22,6 g de 1-(4-acétylaminophényl)-2-chloro-propanone dans 50 ml de pyrrolidine et 20 ml $H_2O$, et porte le mélange résultant au reflux pendant 2 h. On évapore sous vide la pyrrolidine en excès qui n'a pas réagi, puis reprend le résidu d'évaporation huileux par 100 ml HCl 4N. On chauffe au reflux pendant 0,5 h. On évapore sous vide et cristallise dans l'éthanol. On obtient 11,5 g (rendement: 40 %) de CRL 41 222. $F_{inst}=200°C$.

**PREPARATION III**

Obtention du dichlorhydrate de 1-(4-aminophényl)-2-isopropylamino-propanone.

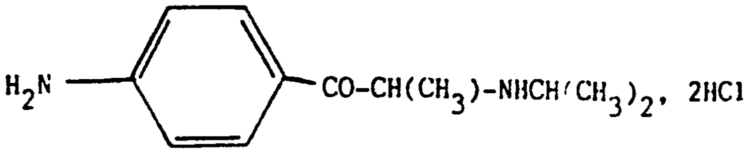

(Exemple 1; N° de Code: CRL 41 153)

On dissout 10 g de chlorhydrate de 1-(4-acétylaminophényl)-2-isopropylamino-propanone dans 100 ml HCl 4N et porte au reflux pendant 0,5 h. Après évaporation sous vide et recristallisation dans le mélange méthanol-acétone (1:1) v/v on obtient 16,7 g (rendement: 60 %) de CRL 41 153. $F_{inst} = 210°C$.

**PREPARATION IV**

Obtention du chlorhydrate de 1-(4-amino-3,5-dichlorophényl)-2-isopropylamino-propanone.

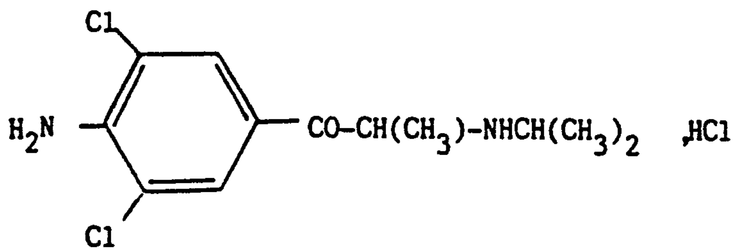

(Exemple 3; N° de Code: CRL 41 194).

A 28 g de dichlorhydrate de 1-(4-aminophényl)-2-isopropylamino-propanone dissous dans 100 ml d'$H_2O$ et maintenu vers 10°C, on ajoute par barbotage 0,2 mole de chlore. Après 2 heures d'agitation on évapore à sec sous vide. Le résidu d'évaporation est repris dans l'acide acétique et par cristallisation on obtient 11 g (rendement: 35 %) de CRL 41 194. $F_{inst} > 260°C$.

**PREPARATION V**

Obtention du dichlorhydrate de 1-(4-aminophényl)-2-morpholino-propanone.

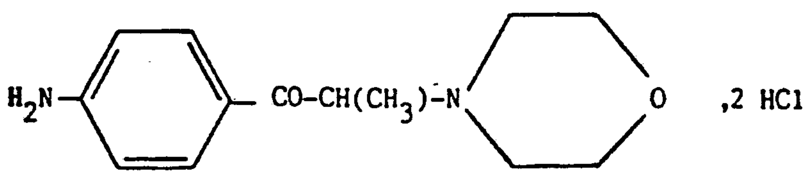

(Exemple 10; N° de Code : CRL 41 237)

On porte au reflux pendant 1 h, une solution de 19 g (0,0608 mole) de chlorhydrate de 1-(4-acétylaminophényl)-2-morpholino-propanone dans 100 ml HCl 4N. On amène à siccité sous pression réduite et on reprend le résidu d'évaporation par du benzène que l'on distille azéotropiquement au moyen d'un Dean-Stark. Le produit attendu cristallise. Par filtration on recueille 11,2 g (rendement: 60 %) de CRL 41 237. $F_{inst} = 210°C$ (avec décomposition).

**PREPARATION VI**

Obtention du dichlorhydrate de 1-(3-aminophényl)-2-pipéridino-propanone.

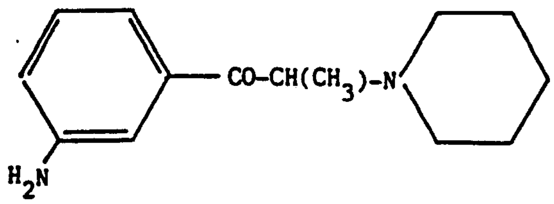
, 2HCl

(Exemple 17; N° de Code: CRL 41 263).

On porte à reflux pendant 1 heure une solution de 9,9 g, (0,0318 mole) de chlorhydrate de 1-(3-acétylaminophényl)-2-pipéridino-propanone dans 50 ml d'acide chlorhydrique 4N. On amène à siccité le milieu réactionnel sous pression réduite et on reprend le résidu par du benzène que l'on distille azéotropiquement au moyen d'un Dean-Stark. Par filtration du précipité formé on obtient 9,8 g (rendement: 100 % environ) de CRL 41 263. $F_{inst}$=200° C (avec décomposition).

**PREPARATION VII**

Obtention du dichlorhydrate de 1-(4-aminophényl)-2-méthylamino-propanone.

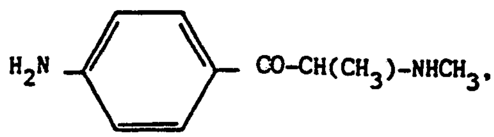
, 2HCl

(Exemple 5; N° de Code: CRL 41 218)

On porte au reflux pendant 0,25 h une solution de 5,5 g (0,021 mole) de chlorhydrate de 1-(4-acétylaminophényl)-2-méthylamino-propanone dans 35 ml d'acide HCl 4N. On amène le milieu réactionnel a siccité, et on reprend le résidu d'évaporation par du benzène que l'on distille azéotropiquement au moyen d'un Dean-Stark. Le précipité formé est purifié par un lavage à chaud dans le méthanol. On obtient 3,8 g (rendement: 72 %) de CRL 41 218. $F_{inst}$=220° C (avec décomposition).

On a résumé ci-après une partie des résultats des essais qui ont été entrepris avec les composés selon l'invention.

A. ESSAIS RELATIFS AU CRL 41 153 (PRODUIT DE L'EXEMPLE 1)

Dans l'étude neuropsychopharmacologique qui suit, le CRL 41 153 en solution dans de l'eau distillée (pH 1,5) a été administré par voie intrapéritonéale sous un volume de 20 ml/kg chez la souris mâle et de 5 ml/kg chez le rat mâle.

1. TOXICITE

Chez la souris mâle la DL-O (dose maximale non mortelle) par voie intrapéritonéale est supérieure à 64 mg/kg et la DL-60 est de l'ordre de environ 128 mg/kg.

II. COMPORTEMENT GLOBAL ET REACTIVITES

Des lots de trois animaux sont observés avant, puis 0,25 h, 0,50 h, 1 h, 2 h, 3 h et 24 h après l'administration de CRL 41 153. On constate:

1. chez la souris

à la dose de 0,5 mg/kg,
- aucun symptôme particulier;

à la dose de 2 mg/kg,
- une excitation de faible intensité 0,5 h après administration,
- des stéréotypies pendant 1 h,
- une augmentation de la réaction de peur (pendant 1 h) et de la réactivité au toucher (2 à 3 h après administration).

à la dose de 8 mg/kg,
- une excitation pendant 3 h,
- une augmentation de la réaction de peur (pendant 3 h) et de la réactivité au toucher (pendant 24 h);

à la dose de 32 mg/kg,
- une excitation et des stéréotypies pendant 3 h,
- une augmentation de la réaction de peur (pendant 2 h) et de la réactivité au toucher (pendant 24 h),

- une faible hyperthermie (+ 1,1° C) 0,5 h après administration, et
- une mydriase modérée;

2. chez le rat

à la dose de 0,25 mg/kg
- aucun symptôme particulier;

à la dose de 1 mg/kg
- une mydriase pendant 1 h atteignant sa valeur maximale 1 h après administration;

à la dose de 4 mg/kg
- une excitation 0,5 h après administration et des stéréotypies pendant 2 h,
- une hyperthermie pendant 3 h maximale 0,5 h après administration (+ 1,9° C),
- une mydriase pendant 2 h atteignant sa valeur maximale 0,5 h après administration;

à la dose de 16 mg/kg
- une excitation 0,5 h après administration,
- des stéréotypies pendant 3 h,
- une augmentation de la réaction de peur pendant 3 h,
- une mydriase pendant 3 h maximale 0,5 h après administration.

III. INTERACTION AVEC L'APOMORPHINE

1. chez la souris

Des lots de 6 souris reçoivent le CRL 41 153 0,5 h avant l'injection sous-cutanée de 1 ou 16 mg/kg d'apomorphine. On observe que aux doses de 2 mg/kg, 8 mg/kg et 32 mg/kg, le CRL 41 153 (qui est hyperthermisant aux dites doses) s'oppose à l'hypothermie induite par l'apomorphine sans modifier les comportements de verticalisation et de stéréotypies.

2. chez le rat

Le CRL 41 153 est administré à des lots de 6 rats 0,5 h avant l'injection sous-cutanée de 0,5 mg/kg d'apomorphine. On observe que, dés la dose de 0,5 mg/kg le CRL 41 153 potentialise les stéréotypies induites par l'apomorphine.

IV. INTERACTION AVEC L'AMPHETAMINE

L'amphétamine (2 mg/kg) est injectée par voie intrapéritonéale à des lots de 6 rats, 30 minutes après l'administration de CRL 41 153. On constate que, aux doses de 1 mg/kg, 4 mg/kg et 16 mg/ kg, le CRL 41 153 potentialise les stéréotypies amphétaminiques en intensité et en durée.

V. INTERACTION AVEC LA RESERPINE

Quatre heures après l'injection intrapéritonéale de 2,5 mg/kg de réserpine, des lots de 6 souris reçoivent le CRL 41 153.

On note que, des la dose de 0,5 mg/kg, le CRL 41 153 s'oppose fortement à l'hypothermie et au ptôsis réserpiniques.

VI. INTERACTION AVEC L'OXOTREMORINE

Le CRL 41 153 est administré à des lots de 6 souris 0,5 h avant l'injection intrapéritonéale de 0,5 m g/kg d'oxotrémorine.

1. Action sur la température

On constate que, aux doses de 2 mg/kg, 8 mg/kg et 32 mg/kg, le CRL 41 153 s'oppose à l'action hypothermisante de l'oxotrémorine.

2. Action sur les tremblements

On constate que, aux doses de 8 mg/kg et 32 mg/kg, le CRL 41 153 antagonise partiellement les tremblements induits par l'oxotrémorine.

3. Action sur les symptômes cholinergiques périphériques

On observe que le CRL 41 153 possède un effet stimulant $\alpha$ -adrénergique modéré.

VII. ACTION SUR LE TEST DES QUATRE PLAQUES, LA TRACTION ET L'ELECTRO-CHOC.

Le test est pratiqué sur des lots de 10 souris, 30 minutes après l'administration de CRL 41 153.

On constate que, aux doses élevées, le SRL 41 153 antagonise les effets convulsivants de l'électrochoc.

VIII. ACTION SUR LA MOTILITE SPONTANEE

0,5 h après avoir reçu le CRL 41 153, les souris (6 par dose, 12 témoins) sont placées en actimètre où leur motilité est enregistrée pendant 30 minutes.

On observe que le CRL 41 153 augmente l'activité motrice spontanée de la souris.

IX. ACTION SUR L'AGRESSIVITE INTERGROUPES

Après avoir séjourné pendant 3 semaines dans chacune des moitiés d'une cage séparées par une cloison opaque, des groupes de 3 souris reçoivent le CRL 41 153. Une demi-heure plus tard, les deux groupes.d'une même cage sont réunis par retrait de la cloison et on note le nombre de combats qui surviennent en 10 minutes.

On constate que, à la dose de 8 mg/kg, le CRL 41 153 diminue le nombre de combats.

X. ACTION VIS-A-VIS DE QUELQUES COMPORTEMENTS PERTURBES PAR DIVERS AGENTS

1. Motilité réduite par habituation à l'enceinte

Après 18 heures de séjour dans les actimètres, les souris (6 par dose, 12 témoins) reçoivent le CRL 41 153. Elles sont aussitôt replacées dans leurs enceintes respectives et, une demi-heure plus tard, on enregistre leur motilité pendant 30 minutes.

On observe que le CRL 41 153 entraîne une reprise nette de l'activité motrice chez la souris habituée à son enceinte.

2. Motilité réduite par agression hypoxique

Une demi-heure après avoir reçu le CRL 41 153, les souris (10 par dose, 20 témoins) sont soumises à une anoxie hypobare aigüe [dépression de 600 mmHg (i.e. environ 8 x $10^4$Pa) en 90 secondes; détente de 45 secondes], puis elles sont placées en actimètre où leur motilité est enregistrée pendant 10 minutes.

On observe que, dès la dose de 0,5 mg/kg, le CRL 41 153 entraîne une amélioration nette de la récupération motrice chez la souris dont la motilité a été déprimée à la suite d'un séjour bref dans une enceinte a pression réduite.

3. Anoxie asphyxique

Des lots de 10 souris reçoivent le CRL 41 153 une demi-heure avant l'administration intrapéritonéale de 32 mg/kg de triiodoéthylate de gallamine (agent curarisant de référence).

On observe que le CRL 41 153, à la dose de 32 mg/kg, diminue le délai d'apparition des convulsions et laisse inchangé le délai d'apparition de la mort, après une anoxie asphyxique provoquée par un curarisant.

XI. INTERACTION AVEC LE BARBITAL

Une demi-heure après l'administration de CRL 41 153, des lots de 10 souris reçoivent une injection intrapéritonéale de barbital (220 mg/kg).

On constate que, aux doses de 0,5 à 32 mg/kg, le CRL 41 153 retarde le délai d'endormissement et diminue la durée du sommeil barbiturique (l'antagonisme est total pour les doses de 8 mg/kg et 32 mg/ kg).

XII. ACTION SUR LE "DESESPOIR COMPORTEMENTAL"

Une demi-heure après avoir reçu le CRL 41 153 des lots de 6 souris sont placés dans un bécher rempli d'eau sur une hauteur de 6 cm. On note la durée totale d'immobilité entre la 2ème et la 6ème minutes suivant l'immersion.

On observe que, aux doses supérieures ou égales à 0,5 mg/kg, le CRL 41 153 diminue la durée de l'immobilité de la souris placée en immersion forcée.

XIII. CONCLUSIONS

Il résulte de l'ensemble des essais neuropsychopharmacologique ci-dessus que le CRL 41 153 présente des effets

- antidépresseurs: antagonisme des hypothermies induites par l'apomorphine, la réserpine ou l'oxotrémorine, et, diminution de la durée de l'immobilité de "désespoir";
- stimulants: excitation chez la souris et le rat, présence de mouvements stéréotypés chez la souris et chez le rat, potentialisation des stéréotypies induites par l'apomorphine et l'amphétamine, augmentation de l'activité motrice, amélioration de la récupération motrice chez la souris dont la motilité a été déprimée à la suite d'un séjour bref dans une enceinte à pression réduite, et, amélioration nette de l'activité motrice chez la souris habituée à son enceinte;
- stimulants $\alpha$-adrénergiques périphériques: mydriase (sans piloérection ni salivation) et antagonisme du ptôsis réserpinique; et
- anticonvulsivants: à dose élevée.

Il s'ensuit que le CRL 41 153 se comporte comme un agent antidépresseur du SNC. L'effet antidépresseur est associé à une composante stimulante nette.

XIV. ESSAIS COMPLEMENTAIRES

1. Par voie orale

Les essais entrepris par administration par voie gastrique, montrent que le CRL 41 153 se présente comme une substance très active selon les tests mis en oeuvre:

- hypermotilité
- diminution du sommeil barbiturique
- antagonisme de l'hypothermie induite par l'apomorphine

D'une manière générale on observe que par voie gastrique les effets antidépresseurs et les effets stimulants et/ou éveillants apparaissent dès la dose de 4 mg/kg. Par voie gastrique, le CRL 41 153 présente donc les mêmes types d'action qu'après administration par voie intrapéritonéale; les effets de type stimulant et/ou éveillant, et les effets de type antidépresseur se développent pour des doses comparables.

2. Etude cardiovasculaire

On constate que le CRL 41 153 augmente chez le chien anesthésié le débit de l'artère fémorale et le débit de l'artère vertébrale, aux doses de 2,5 à 5 mg/kg per os, L'augmentation des débits fémoral et vertébral est

supprimée par le propanolol. On observe également que le CRL 41 153 ne modifie pas les effets de l'isoprénaline sur la pression artérielle, et qu'il présente un effet stimulant α-adrénergique modéré. La bradycardie réflexe à l'hypertension est transformée en tachycardie (l'origine de cet effet serait de nature atropinique ou ganglioplégique).

3. Différence avec l'amphétamine.

Le CRL 41 153 se différencie de l'amphétamine au niveau du mécanisme de ses effets stéréotypigènes et de son action au niveau du système dopaminergique nigro-strié (explorée par l'interaction avec l'alpha-méthyl-tyrosine - celle-ci inhibe totalement les stéréotypies amphétaminiques mais les diminue seulement dans le cas du CRL 41 153).

B. ESSAIS RELATIFS AU CRL 41 178 (PRODUIT DE L'EXEMPLE 2)

L'étude neuropsychopharmacologique du CRL 41 178 entreprise selon les modalités décrites ci-dessus pour le CRL 41 153, montre que ledit CRL 41 178 présente par voie intrapéritonéale des effets

- stimulants nets: excitation et hyper-réactivité chez la souris et le rat, présence de mouvements stéréotypés et potentialisation des effets de l'apomorphine et de l'amphétamine chez le rat, antagonisme du sommeil barbiturique, augmentation de l'activité motrice, reprise de l'activité chez le souris habituée à son enceinte, amélioration de la récupération motrice chez la souris dont l'activité a été déprimée à la suite d'une hypoxie hypobare;
- antidépresseurs, plus modestes: antagonisme des hypothermies induites par la réserpine, l'oxotrémorine ou l'apomorphine, diminution de la durée d'immobilité de "désespoir";
- stimulants α-adrénergiques modérés: mydriase (sans piloérection ni salivation) et léger antagonisme du ptôsis réserpinique;
- anticonvulsivants: a doses élevées, antagonisme des effets convulsivants de l'électrochoc.

Le CRL 41 178 se comporte sur l'ensemble de ces essais comme un stimulant, dès les plus faibles doses. Les effets stimulants sont associés à une composante antidépressive.

Par voie gastrique, le CRL 41 178 présente les mêmes types d'action qu'après administration intrapéritonéale. Il convient de signaler que les effets antidépresseurs sont, semble-t-il, mieux révélés par voie orale que par voie intrapéritonéale.

Différence avec l'amphétamine

Les stéréotypies amphétaminiques, inhibées totalement après blocage par l'alpha-méthyl-tyrosine de la synthèse des catécholamines, semblent dépendre essentiellement d'un pool de catécholamines nouvellement synthétisées (dopamine probablement). Ce pool semble impliqué, au moins partiellement, dans le mécanisme qui sous-tend les stéréotypies induites par le CRL 41 178.

Le CRL 41 178 se rapproche de l'amphétamine, notamment par la présence de mouvements stéréotypés et par l'existence d'une toxicité particulière chez les souris groupées, mais il se différencie de l'amphétamine au niveau du mécanisme des effets stéréotypigènes.

C. ESSAIS RELATIFS AU CRL 41 194 (PRODUIT DE L'EXEMPLE 3)

- ESSAIS NEUROPSYCHOPHARMACOLOGIQUES

Les essais neuropsychopharmacologiques ont été entrepris selon les modalités opératoires décrites ci-dessus pour le CRL 41 153. Dans ces essais et sauf indications contraires le CRL 41 194 a été administré en solution dans de l'eau distillée (pH 4,5) par voie intrapéritonéale sous un volume de 20 ml/kg chez la souris mâle et de 5 ml/kg chez le rat mâle.

I. TOXICITE

Par voie intrapéritonéale chez la souris mâle la DL-O est supérieure à 32 mg/kg et la DL-50 est égale à 86 mg/kg.

II. COMPORTEMENT GLOBAL ET REACTIVITES

On observe

1. chez la souris

aux doses de 0,25 mg/kg et 1 mg/kg:

- une faible excitation 24 h après administration;

à la dose de 4 mg/kg

- une faible excitation et une augmentation de la réactivité au toucher, environ 24 h après administration;

à la dose de 16 mg/kg

- une faible excitation 24 h après administration,
- une augmentation de la réactivité au toucher pendant 24 h,
- une dyspnée pendant 3 h,
- une mydriase durant 2 h (maximale 1 h après administration),
- une dilatation des veines de la queue pendant 2 h;

2. chez le rat

à la dose de 0,125 mg/kg

- une mydriase pendant 2 h (maximale 0,5 h après administration);

à la dose de 0,5 mg/kg

- une dyspnée pendant 0,5 h,

à la dose de 2 mg/kg

- une mydriase maximale 0,5 h après administration,
- une dyspnée pendant 2 h,

à la dose de 8 mg/kg

- une piloérection pendant 1 h,
- une mydriase pendant 2 h (maximale 0,5 h après administration),
- une dyspnée pendant 2 h.

III. INTERACTION AVEC L'APOMORPHINE

On constate que le CRL 41 194 administré par voie gastrique s'oppose dès la dose de 2 mg/kg à l'hypothermie induite chez la souris par l'injection sous-cutanée de 16 mg/kg d'apomorphine sans modifier le comportement de verticalisation et les stéréotypies.

IV. ACTION SUR LA MOTILITE SPONTANEE

A la plus forte dose utilisée par voie gastrique (32 mg/kg), le CRL 41 194 diminue faiblement l'activité motrice spontanée de la souris.

V. INTERACTION AVEC LE BARBITAL

Par voie gastrique, à la plus forte dose utilisée (32 mg/kg), le CRL 41 194 diminue la durée du sommeil barbiturique chez la souris.

VI. RECHERCHE DE MOUVEMENTS STEREOTYPES

Immédiatement après l'administration du CRL 41 194 par voie intrapéritonéale ou d'amphétamine, des lots de 6 rats sont placés dans des boîtes en plexiglass (20x10x10 cm). Les mouvements stéréotypés sont côtés de 0 à 3 toutes les 10 minutes, jusqu'à leur disparition.

Le CRL 41 194, à la plus forte dose utilisée (16 mg/kg), provoque l'apparition de mouvements stéréotypés. L'intensité de l'effet obtenu avec 16 mg/kg est légèrement inférieure à celle obtenue avec 2 mg/kg d'amphétamine.

VII. RECHERCHE D'UNE TOXICITE PARTICULIERE CHEZ LES SOURIS GROUPEES

Aussitôt après l'administration par voie intrapéritonéale de CRL 41 194, les souris groupées par lots de 10, sont placées dans des boîtes en plexiglass (20 x 10 x 10 cm). Le nombre d'animaux morts est noté après 1 heure, 2 h, 3 h, 4 h et 24 heures. La toxicité du CRL 41 194 est déterminée, dans les mêmes conditions, sur des lots de souris isolées, c'est-à-dire placées à raison d'une par boîte.

La DL-50 du CRL 41 194 est égale à:

. 86 mg/kg chez les souris isolées,

. 27 mg/kg chez les souris groupées.

Le rapport DL-50 souris isolées/DL-50 souris groupées, est égal à 3,19 (statistiquement significatif).

Le CRL 41 194 est donc plus toxique chez les souris groupées que chez les souris isolées. A titre de comparaison, le rapport DL-50 souris isolées/DL-50 souris groupées, dans les mêmes conditions est:

. voisin de 8 pour l'amphétamine,

. voisin de 4 pour la nomifensine et la benzphétamine,

. voisin de 6 pour le méthylphénidate.

VIII. CONCLUSION

Dans ces essais le CRL 41 194, administré par voie intrapéritonéale ou gastrique, se présente comme un agent antidépresseur. Par voie intrapéritonéale on constate que les effets antidépresseurs se manifestent en même temps que l'apparition de mouvements stéréotypés (d'intensité modérée).

ETUDE CARDIOVASCULAIRE

Deux chiens (poids moyen: 14, 1 kg) anesthésiés au nembutal reçoivent le CRL 41 194 par voie intraduodénale, aux doses successives de 0,1 mg/kg, 0,5 mg/kg, 1 mg/kg, 2,5 mg/kg, 5 mg/kg et 10 mg/kg (en solution dans du soluté physiologique).

On mesure la pression artérielle, la fréquence cardiaque, le débit artériel fémoral, le débit artériel vertébral, la température rectale. On observe la coloration de la peau et la coloration de la bile recueillie par cathétérisation du cholédoque après ligature du cystique.

On constate que le CRL 41 194 augmente le débit vertébral dès la dose de 0,1 mg/kg I.D.

Le débit fémoral augmente à 0,5 mg/kg en même temps que la fréquence cardiaque. On observe une baisse de la pression artérielle diastolique à 5 mg/kg, ainsi qu'une augmentation de la pression artérielle différentielle, dès les premières doses. Les deux chiens ont eu une stimulation respiratoire, et les températures rectale et cutanée se sont élevées.

Les effets de l'isoprénaline, testés après la dose cumulée de 19,1 mg/kg I.D., ne sont pas modifiés en ce qui concerne la fréquence cardiaque, et sont diminués en ce qui concerne la pression artérielle.

A 10 µg/kg d'isoprénaline, la pression artérielle diastolique passe de 126 mmHg (soit environ 1,67 x $10^4$Pa) à

64 mmHg (soit environ 8,5 x $10^3$Pa) après CRL 41 194, au lieu de 154 mmHg (soit environ 2,05 x $10^4$Pa) à 38 mmHg (soit environ 5,06 x $10^3$Pa) en témoin, et la fréquence cardiaque passe de 212 bat/min. aprés CRL 41 194 au lieu de 152 bat/min. à 260 bat/min. en valeur témoin.

L'hypertension induite par la noradrénaline est légèrement diminuée par le CRL 41 194. A 2 µg/kg de noradrénaline la pression artérielle systolique passe de 208 mmHg (soit environ 2,77 x $10^4$Pa) à 266 mmHg (soit environ 3,54 x $10^4$Pa) au lieu de 178 mmHg (soit environ 2,37 x $10^4$Pa) à 312 mmHg (soit environ 4,15 x $10^4$Pa) en témoin.

ETUDE IMMUNOLOGIQUE

Le CRL 41 194 présente une activité sur l'immunité cellulaire et humorale selon le test dit des cellules formant des plages de lyse décrit par A.J. CUNNINGHAM et al. ("Further improvements in the plaque technique for detecting single antibody forming cells"), Immunology 14, pages 599-601, (1968), et selon la mesure de l'intensité de l'hypersensibilité retardée aux globules rouges de mouton décrite par T.E. MILLER et al. ("Immunopotentiation with BCG II modulation of the response to sheep blood cells"), Journal of the National Cancer Institute 51 (N° 5), pages 1669-1676, (1973). Il résulte des essais correspondants que le CRL 41 194 se comporte comme un agent immuno-modulateur.

ETUDE CLINIQUE

En clinique, chez l'homme, le CRL 41 194 s'est avéré très actif en tant que vasodilatateur dans le traitement des maladies liées à la circulation sanguine au niveau vasculaire.

Par ailleurs il a donné également chez l'homme d'excellents résultats dans le traitement des dépressions à la dose de 3 x 5 mg par jour, notamment sous forme de comprimés ou gélules renfermant chacun 5 mg de CRL 41 194, à raison de trois prises quotidiennes.

D. ESSAIS RELATIFS AU CRL 41 213 (PRODUIT DE L'EXEMPLE 4)

L'étude neuropsychopharmacologique a été entreprise selon les modalités décrites ci-dessus pour le CRL 41 153, le CRL 41 213 étant administré en solution dans de l'eau distillée par voie intrapéritonéale sous un volume de 20 ml/kg chez la souris mâle et un volume de 5 ml/kg chez le rat mâle, le pH de la solution injectée variant en fonction de la concentration en CRL 41 213 selon les indications données dans le tableau II ci-après.

**TABLEAU II**

Variation du pH de solutions dans l'eau distillée de certains composés selon l'invention.

| PRODUIT | CONCENTRATION (g/l) | pH |
|---|---|---|
| | 50 | 1,0 |
| | 25 | 1,5 |
| | 3,2 | 2,0 |
| | 1,6 | 2,5 |
| Ex 4 (CRL 41 213) | 0,8 | 3,0 |
| | 0,4 | 3,5 |
| | 0,2 | 4,0 |
| | ⩽ 0,1 | 5,0 |
| | 50 | 4,0 |
| Ex 5 (CRL 41 218) | 13 | 4,5 |
| | 6 | 5,0 |
| | ⩽ 3 | 5,5 |
| | 50 | 4,0 |
| Ex 6 (CRL 41 221) | 13 | 4,5 |
| | 6 | 5,0 |
| | ⩽ 3 | 5,5 |

| | | |
|---|---|---|
| | 12,8 | 1,5 |
| | 6,4 | 2,0 |
| | 3,2 | 2,5 |
| | 1,6 | 3,0 |
| Ex 7 (CRL 41 222) | 0,8 | 3,5 |
| | 0,4 | 4,0 |
| | 0,2 | 4,5 |
| | 0,1 | 5 |
| | ⩽ 0,05 | 5,5 |
| | 50 | 1,5 |
| | 13 | 2,0 |
| | 3 | 2,5 |
| Ex 8 (CRL 41 225) | 2 | 3,0 |
| | 0,8 | 3,5 |
| | 0,4 | 4,0 |
| | 0,2 | 4,5 |
| | 0,1 | 5,0 |
| | ⩽ 0,05 | 5,5 |
| | 50 | 1,0 |
| | 12,8 | 1,5 |
| | 6,4 | 2,0 |
| | 3,2 | 2,5 |
| Ex 9 (CRL 41 233) | 1,6 | 3,0 |
| | 0,8 | 3,5 |
| | 0,4 | 4,0 |
| | 0,2 | 4,5 |
| | 0,1 | 5,0 |
| | ⩽ 0,05 | 5,5 |
| | 50 | 1,0 |
| | 25 | 1,5 |
| | 6,4 | 2,0 |
| | 3,2 | 2,5 |
| | 1,6 | 3,0 |
| Ex 10 (CRL 41 237) | 0,8 | 3,5 |
| | 0,4 | 4,0 |
| | 0,2 | 4,5 |
| | 0,1 | 5,0 |
| | ⩽ 0,05 | 5,5 |
| | 50 | 1,0 |
| | 26 | 1,5 |
| | 6 | 2,0 |
| | 3 | 2,5 |
| Ex 11 (CRL 41 241) | 2 | 3,0 |
| | 1 | 3,5 |
| | 0,5 | 4,5 |
| | 0,3 | 5,0 |
| | ⩽ 0,05 | 5,5 |

| | | |
|---|---|---|
| | 50 | 1,0 |
| | 25 | 1,5 |
| | 6,4 | 2,0 |
| | 3,2 | 2,5 |
| Ex 12 (CRL 41 243) | 0,8 | 3,5 |
| | 0,4 | 4,0 |
| | 0,2 | 4,5 |
| | 0,1 | 5,0 |
| | ⩽ 0,05 | 5,5 |
| | 12,8 | 2,5 |
| | 6,4 | 3,0 |
| | 1,6 | 3,5 |
| Ex 13 (CRL 41 246) | 0,8 | 4,0 |
| | 0,4 | 4,5 |
| | 0,1 | 5,0 |
| | ⩽0,05 | 5,5 |
| | 50 | 1,5 |
| | 6,4 | 2,0 |
| | 3,2 | 2,5 |
| | 1,2-0,8 | 3,0 |
| | 0,4 | 3,5 |
| Ex 14 (CRL 41 248) | 0,2 | 4,0 |
| | 0,1 | 4,5 |
| | 0,05 | 5,0 |
| | ⩽0,025 | 5,5 |

I. TOXICITE

Par voie intrapéritonéale, chez la souris mâle, la DL-O du CRL 41 213 est supérieure ou égale à 128 mg/kg, et la DL-100 est inférieure ou égale à 256 mg/kg.

II. COMPORTEMENT GLOBAL ET REACTIVITES

1. chez la souris

à la dose de 1 mg/kg
- sédation modérée 0,5 h après administration;

à la dose de 4 mg/kg
- excitation modérée 0,5 h après administration,
- piloérection pendant 0,25 h;

à la dose de 16 mg/kg
- excitation pendant plus de 3 h avec présence de stéréotypies pendant environ 2 h à partir de la 1ère heure qui suit l'administration,
- augmentation de la réaction de peur, de la réactivité au toucher, et de l'agressivité pendant plus de 3 h,
- salivation, lacrymation et exophtalmie pendant 1 h,
- polypnée; et,

à la dose de 64 mg/kg.
- convulsions cloniques pendant 0,5 h,
- excitation pendant plus de 3 h, avec présence de stéréotypies,
- augmentation de la réaction de peur et de la réactivité au toucher de 2 h à plus de 3 h,
- hypothermie pendant 1 h (valeur maximale: - 2,5° S),
- mydriase pendant plus de 3 h,
- polypnée;

2. chez le rat

à la dose de 0,5 mg/kg
- mydriase pendant 1 h;

à la dose de 2 mg/kg
- excitation avec présence de stéréotypies,

- augmentation de la réaction de peur, de la réactivité au toucher et au bruit pendant 2 h,

à la dose de 8 mg/kg
- excitation avec présence de stéréotypies,
- augmentation de la réaction de peur, de la réactivité au toucher et au bruit pendant 3 h,
- mydriase pendant plus de 3 h; et,

à la dose de 32 mg/kg
- excitation avec présence de stéréotypies très intenses,
- augmentation de la réaction de peur, de la réactivité au toucher et au bruit pendant 3 h,
- exophtalmie de 0,5 h à 3 h,
- mydriase pendant 3 h,
- polypnée.

III. RECHERCHE DE MOUVEMENTS STEREOTYPES

Des lots de 6 rats reçoivent une injection intrapéritonéale de CRL 41 213, d'eau distillée, ou d'amphétamine immédiatement avant d'être placés dans des cages de dimensions réduites où leur comportement stéréotypé est coté toutes les 10 minutes jusqu'à extinction de l'effet.

Dès la dose de 2 mg/kg, le CRL 41 213 provoque, chez le rat, l'apparition de mouvements stéréotypés, dont l'intensité est sensiblement comparable à celle obtenue avec 2 mg/kg d'amphétamine. L'augmentation des doses conduit à une augmentation de l'intensité et de la durée de ces stéréotypies.

IV. INTERACTION AVEC L'APOMORPHINE.

1. chez la souris

On observe que, aux doses de 1 mg/kg, 4 mg/kg, 16 mg/kg et 64 mg/kg le CRL 41 213 s'oppose fortement à l'hypothermie induite par l'apomorphine chez la souris. A la plus forte dose étudiée (64 mg/kg) le SRL 41 213 diminue modérément le comportement de verticalisation sans modifier les stéréotypies induites par l'apomorphine.

2. chez le rat

On constate que, dès la dose de 2 mg/kg, le CRL 41 213 provoque l'augmentation de l'index des stéréotypies induites par l'apomorphine. Cette augmentation est importante en intensité et en durée, aux doses de 8 mg/kg et de 32 mg/kg de CRL 41 213.

V. INTERACTION AVEC L'AMPHETAMINE

On constate que, dès la dose de 2 mg/kg, le CRL 41 213 potentialise les stéréotypies amphétaminiques. L'intensité et la durée de la potentialisation croissent avec la dose.

VI. INTERACTION AVEC LA RESERPINE

On observe que, aux doses de 1 mg/kg et de 4 mg/kg, le CRL 41 213 s'oppose à l'action hypothermisante de la réserpine ainsi qu'au ptôsis réserpinique, et que, à dose plus faible (0,25 mg/kg) aucun effet n'est détecté.

VII. INTERACTION AVEC L'OXOTREMORINE

1. Action sur la température

Dès la dose de 4 mg/kg le CRL 41 213 s'oppose à l'action hypothermisante de l'oxotrémorine. A la plus faible dose étudiée (1 mg/kg) cet effet est à peine décelable.

2. Action sur les tremblements

Aux plus fortes doses (16 et 64 mg/kg) le CRL 41 213 diminue l'intensité des tremblements dûs à l'oxotrémorine.

3. Action sur les symptômes cholinergiques périphériques

Le CRL 41 213 laisse inchangés les signes de stimulation cholinergiques périphériques produits par l'oxotrémorine.

VIII. ACTION SUR LE TEST DES QUATRE PLAQUES, LA TRACTION ET L'ELECTROCHOC

Aux doses de 1 mg/kg, 4 mg/kg et 16 mg/kg le CRL 41 213 entraîne une augmentation du nombre de passages punis. Cette augmentation n'est pas présente à la plus forte dose utilisée (64 mg/kg). A forte dose, le CRL 41 213 entraîne une incapacité motrice (64 mg/kg) et s'oppose aux effets convulsivants de l'électrochoc (16 à 64 mg/kg). A la plus forte dose utilisée (64 mg/kg) la présence de convulsions cloniques est susceptible de constituer une perturbation au niveau du nombre de passages punis, du test de la traction et des convulsions induites par l'électrochoc.

IX. ACTION SUR LA MOTILITE SPONTANEE

Aux doses de 1 mg/kg, 4 mg/kg et 16 mg/kg le CRL 41 213 entraîne une importante augmentation de l'activité motrice spontanée de la souris. A la plus forte dose étudiée (64 mg/kg) le CRL 41 213 provoque des convulsions cloniques et des stéréotypies, qui peuvent interférer avec l'activité motrice spontanée de la souris.

X. ACTION SUR L'AGRESSIVITE INTERGROUPES

On observe que le CRL 41 213 ne modifie pas l'agressivité intergroupes chez la souris.

XI. ACTION VIS-A-VIS DE QUELQUES COMPORTEMENTS PERTURBES PAR DIVERS AGENTS

1. Motilité réduite par habituation à l'enceinte

On observe que le CRL 41 213 aux doses de 4 mg/kg, 16 mg/ kg, et 64 mg/kg provoque une reprise de l'activité motrice chez la souris habituée à son enceinte.

2. Motilité réduite par agression hypoxique

Dès 1 mg/kg le CRL 41 213 entraîne une amélioration nette de la récupération motrice chez la souris dont l'activité a été déprimée à la suite d'un bref séjour dans une enceinte à pression réduite.

3. Anoxie asphyxique

On constate que le CRL 41 213 ne modifie pas le délai d'apparition des convulsions et, que, à la dose de 64 mg/kg, il retarde modérément l'apparition de la mort consecutive à une anoxie asphyxique provoquée par un curarisant de référence, le triiodoéthylate de gallamine.

XII. INTERACTION AVEC LE BARBITAL

On observe que, dès la dose de 0,5 mg/kg le CRL 41 213 diminue la durée du sommeil barbiturique. L'antagonisme est total pour des doses égales ou supérieures à 4 mg/kg.

XIII. ACTION SUR LE "DESESPOIR COMPORTEMENTAL"

On constate que dès la dose de 0,5 mg/kg le CRL 41 213 diminue nettement la durée d'immobilité de "désespoir". Set antagonisme est total aux doses de 4 mg/kg, 16 mg/kg, et 64 mg/kg de CRL 41 213.

XIV. CONCLUSION

Eu égard aux résultats donnés ci-dessus, le CRL 41 213 présente dans son profil neuropsychopharmacologique des effets

- stimulants

. excitation avec hyper-réactivité chez la souris et le rat,

. hyperactivité (augmentation de l'activité motrice, reprise de l'activité motrice chez le souris habituée à son enceinte, amélioration de la récupération motrice chez la souris ayant subi une agression hypoxique et augmentation du nombre de passages punis au test des 4 plaques),

. antagonisme du sommeil barbiturique,

. présence de mouvements stéréotypés chez la souris et le rat et potentialisation des stéréotypies induites par l'amphétamine et l'apomorphine;

- antidépresseurs:

. antagonisme des hypothermies induites par l'apomorphine, la réserpine et l'oxotrémorine,

. diminution de l'immobilité de "dèsespoir";

>- reflétant une stimulation α-adrénergique périphérique:

. antagonisme du ptôsis réserpinique,

. antagonisme des tremblements provoqués par l'oxotrémorine,

. mydriase,

. présence de salivation, lacrymation, exophtalmie.

De plus, à doses élevées mais non toxiques, le CRL 41 213 entraîne l'apparition de convulsions cloniques mais s'oppose aux effets convulsivants de l'électrochoc.

En bref selon l'ensemble des tests précités, le CRL 41 213 se comporte comme une substance douée de propriétés antidépressive; stimulantes et éveillantes. Ces propriétés se manifestent à des doses relativement faibles.

E. ESSAIS RELATIFS AU CRL 41 218 (PRODUIT DE L'EXEMPLE 5)

Les essais neuropsychopharmacologiques entrepris selon les modalités opératoires décrites ci-dessus pour le CRL 41 153, montrent que le CRL 41 218 présente des effets

- antidépresseurs:

. antagonisme des hypothermies induites par l'apomorphine, la réserpine et l'oxotrémorine,

. diminution de la durée d'immobilité de "désespoir";

- stimulants:

. excitation chez le souris et le rat avec présence de mouvements stéréotypés,

. potentialisation des stéréotypies induites par l'apomorphine et l'amphétamine,

. antagonisme net du sommeil barbiturique,

. hyperactivité (augmentation de l'activité motrice, amélioration de la récupération motrice chez le souris dont la motilité a été déprimée à la suite d'un séjour bref dans une enceinte à pression réduite, reprise de l'activité motrice chez la souris habituée à son enceinte);

- stimulant α-adrénergique:

. mydriase,

. salivation,

. antagonisme du ptôsis réserpinique;

- anticonvulsivants:

. antagonisme à forte dose des effets convulsivants de l'électrochoc.

F. ESSAIS RELATIFS AU CRL 41 221 (PRODUIT DE L'EXEMPLE 6)

I. ETUDE CARDIOVASCULAIRE

4 chiens (poids moyen: 14 kg) anesthésiés au nembutal reçoivent le CRL 41 221 par voie intraduodénale aux doses successives de 0,1 mg/kg, 0 5 mg/kg, 1 mg/kg, 2,5 mg/kg, 5 mg/kg et 10 mg/kg. On mesure la pression artérielle, la fréquence cardiaque, le débit artériel fémoral, le débit artériel vertébral, la température rectale. On observe la coloration de la peau.

On constate que le CRL 41 221 est tachycardisant à partir de 2,5 mg/kg, que le débit fémoral augmente à partir de cette même dose de façon importante, que le débit vertébral augmente à la dose de 0,5 mg/kg. A 5 mg/kg on observe une diminution de la pression artérielle diastolique, l'hypotension se manifeste à 10 mg/kg.

On observe que les températures rectale et cutanée s'élèvent progressivement et que la respiration est stimulée.

On note que les effets de l'isoprénaline testés après la dose cumulée de 19,1 mg/kg de CRL 41 221 ne sont pas modifiés sur la pression artérielle diastolique et qu'ils sont légèrement diminués sur la fréquence cardiaque.

A 3 µg/kg d'isoprénaline la fréquence cardiaque passe de 212 bat/min. à 262 bat/min. après CRL 41 221, au lieu de 181 bat/min. à 272 bat/min. en valeur témoin.

L'hypertension à la noradrénaline est légèrement diminuée. A 2 µg/kg de noradrénaline la pression artérielle systolique passe de 158 mmHg (soit environ $2,10 \times 10^4$Pa) a 266 mmHg (soit environ $3,01 \times 10^4$Pa) au lieu de 183 mmHg (soit environ $2,44 \times 10^4$Pa) à 302 mmHg (soit environ $4,02 \times 10^4$Pa) en témoin.

L'injection chez 2 chiens d'aténolol diminue les effets du CRL 41 221, l'injection de propanolol n'augmente pas les effets β.

Il en résulte que le SRL 41 221 est un agent vasodilatateur.

II. ETUDE NEUROPSYCHOPHARMACOLOGIQUE

Les essais entrepris selon les modalités opératoires décrites ci-dessus pour le CRL 41 153 montrent que le CRL 41 221 présente des effets

- antidépresseurs:

. antagonisme des hypothermies provoquées par la réserpine, l'oxotrémorine et l'apomorphine,

. une diminution de la durée de l'immobilité de la souris placée en immersion forcée (qui peut être en relation avec la composante stimulante);

- stimulants:

. excitation avec hyper-réactivité chez la souris et le rat,

. augmentation de l'activité motrice spontanée chez la souris, avec reprise de l'activité motrice chez la souris habituée à son enceinte et amélioration de la récupération motrice chez la souris placée en hypoxie aigüe,

. augmentation modérée du nombre de passages punis au test des 4 plaques chez la souris (à forte dose),

. diminution de la durée du sommeil barbiturique,

. présence de mouvements stéréotypés chez la souris et le rat,

. potentialisation des stéréotypies induites par l'apomorphine et amphétamine chez le rat.

Par ailleurs, le CRL 41 221 montre à fortes doses:

. une composante de stimulation α-adrénergique objectivée par une diminution du ptôsis réserpinique, une mydriase et la salivation chez la souris et le rat;

. une diminution de l'agressivité et du délai d'apparition des convulsions et de la mort consécutives à une anoxie asphyxique provoquée par un curarisant;

. une diminution des effets convulsivants de l'électrochoc.

Le CRL 41 221 se présente donc comme une substance douée de propriétés antidépressives, avec une importante composante stimulante.

III. ETUDE IMMUNOLOGIQUE

Les essais immunologiques montrent que le CRL 41 221 présente un effet immuno-modulateur, et augmente l' immunité cellulaire.

G. ESSAIS RELATIFS AU CRL 41 222 (PRODUIT DE L'EXELMPLE 7)

I. ETUDE CARDIOVASCULAIRE

Le CRL 41 222, administré par voie intraduodénale à 4 chiens anesthésiés, augmente les débits fémuraux et vertébraux de façon importante et régulière. L'effet est proportionnel à la dose. Cette augmentation des débits s'accompagne d'une tachycardie. Une action hypotensive apparait à 20 mg/kg par une baisse de la pression artérielle diastolique. Les températures rectale et cutanée s'élèvent. Une stimulation respiratoire est observée chez tous les animaux.

Le CRL 41 222 augmente modérément l'hypertension à la noradrénaline sans modifier les effets de l'isoprénaline. Le propanolol, injecté en fin d'expérimentation, montre que le CRL 41 222 agit par stimulation des récepteurs ß cardiaques et vasculaires.

II. ETUDE NEUROPSYCHOPHARMACOLOGIQUE

Les essais entrepris selon les modalités opératoires décrites ci-dessus pour le CRL 41 153, montrent que le

CRL 41 222 présente des effets

- antidépresseurs:

. antagonisme des hypothermies induites par l'apomorphine, la réserpine et l'oxotrémorine,

. diminution de la durée d'immobilité de "désespoir";

- stimulants:

. excitation chez la souris et le rat, avec présence de mouvements stéréotypés,

. potentialisation des stéréotypies induites par l'apomorphine et l'amphétamine,

. antagonisme net du sommeil barbiturique,

. augmentation de l'activité motrice, amélioration de la récuperation motrice chez la souris dont la motilité a été déprimée à la suite d'un séjour bref dans une enceinte à pression réduite, reprise de l'activité motrice chez la souris habituée à son enceinte;

- stimulants α-adrénergiques périphériques:

. mydriase,

. salivation, lacrymation,

. antagonisme du ptôsis réserpinique,

. antagonisme des tremblements induits par l'oxotrémorine.

Le CRL 41 222 se comporte donc comme une substanse douée de propriétés antidépressives, stimulantes et éveillantes. Sa DL-50 chez la souris mâle est de 87 mg/kg I.P.

III. ETUDE CLINIQUE

Chez l'homme, le CRL 41 222 s'est révélé être un bon agent vasodilatateur périphérique à la dose quotidienne de 2 x 10 mg, sous forme de comprimés ou gélules renfermant chacun 10 mg de CRL 41 222, à raison de 2 prises par jour.

H. ESSAIS RELATIFS AU CRL 41 225 (PRODUIT DE L'EXEMPLE 8)

Les essais neuropsychopharmacologiques entrepris selon les modalités opératoires décrites ci-dessus pour le CRL 41 153, montrent que le CRL 41 225 présente des effets:

- antidépresseurs:

. antagonisme des hypothermies induites par l'apomorphine, la réserpine et l'oxotrémorine,

. diminution de la durée d'immobilité de "désespoir";

- stimulants:

. excitation avec hyper-réactivité chez la souris et le rat,

. augmentation de l'activité motrice spontanée chez la souris, reprise de l'activité motrice chez la souris habituée à son enceinte, amélioration de la récupération motrice chez la souris dont la motilité a été déprimée par un bref séjour dans une enceinte sous pression réduite,

. augmentation du nombre de passages punis au test des 4 plaques chez la souris,

. diminution de la durée du sommeil barbiturique,

. présence de mouvements stéréotypés chez la souris et le rat,

. potentialisation des stéréotypies induites par l'amphétamine et l'apomorphine chez le rat.

Par ailleurs, le CRL 41 225 montre:

. une diminution de l'intensité des tremblements provoqués par l'oxotrémorine,

. une composante de stimulation α-adrénergique objectivée par une diminution du ptôsis réserpinique chez la souris et, à forte dose, de la mydriase et de la salivation,

. une diminution du délai d'apparition des convulsions et de la mort consécutives à une anoxie asphyxique provoquée par un curarisant chez la souris,

. une diminution du nombre de combats au test de l'agressivité chez la souris, à forte dose,

. une diminution des effets convulsivants de l'électrochoc, à forte dose.

De plus à côté des propriétés antidépressives et stimulantes, on constate que le CRL 41 225 présente des propriétés vasodilatatrices bénéfiques sur le plan clinique.

I. ESSAIS RELATIFS AU CRL 41 233 (PRODUIT DE L'EXEMPLE 9)

I. ETUDE CARDIOVASCULAIRE

Le CRL 41 233, administré par voie intraduodénale au chien anesthésié, augmente les débits fémoraux et vertébraux à partir de la dose de 5 mg/kg, l'effet tachycardisant apparaissant à 10 mg/ kg. La température rectale augmente fortement.

II. ETUDE NEUROPSYCHOPHARMACOLOGIQUE

Selon les résultats des essais entrepris en utilisant les modalités opératoires décrites pour le CRL 41 153, le CRL 41 233 présente des effets

- antidépresseurs:

. antagonisme des hypothermies induites par l'apomorphine, la réserpine et l'oxotrémorine,

. diminution de la durée d'immobilité de "désespoir"

- stimulants:

. excitation avec hyper-réactivité chez la souris et le rat,

. augmentation de l'activité motrice spontanée chez la souris, reprise de l'activité motrice chez la souris habituée à son enceinte, amélioration de la récupération motrice chez la souris dont la motilité a été déprimée

par un bref séjour dans une enceinte sous pression réduite,

. diminution de la durée du sommeil barbiturique,

. présence de mouvements stéréotypés chez la souris et le rat,

. potentialisation des stéréotypies induites par l'amphétamine et l'apomorphine chez le rat.

III. CONCLUSIONS

Le CRL 41 233 se comporte comme une substance ayant, d'une part, des propriétés vasodilatatrices, et, d'autre part, des propriétés antidépressives et stimulantes.

J. ESSAIS RELATIFS AU CRL 41 237 (PRODUIT DE L'EXEMPLE 10)

I. ETUDE CARDIOVASCULAIRE

Le CRL 41 237, administré par voie introduodénale à un chien anesthésié se montre légèrement tachycardisant à 20 mg/kg. Aucune action n'a été observée sur la pression artérielle, le débit fémoral et le débit vertébral, par voie introduodénale. En revanche, par voie intraveineuse une dose supplémentaire de 10 mg/kg de CRL 41 237 a entraîné une augmentation du débit fémoral. La température rectale s'élève progressivement, la température cutanée augmente fortement.

Le CRL 41 237 ne modifie pas les effets de l'isoprénaline, mais augmente nettement l'hypertension à la noradrénaline.

II. ETUDE NEUROPSYCHOPHARMACOLOGIQUE

Les essais neuropsychopharmacologiques entrepris selon les modalités décrites ci-dessus pour le CRL 41 153, montrent que le CRL 41 237 présente des effets

- antidépresseurs:

. antagonisme des hypothermies induites par l'apomorphine, la réserpine et l'oxotrémorine,

. diminution de la durée d'immobilité de "désespoir';

- stimulants:

. excitation avec hyper-réactivité chez la souris et le rat,

. augmentation de l'activité motrice spontanée chez la souris, reprise de l'activité motrice chez la souris habituée à son enceinte, amélioration de la récupération motrice chez la souris dont la motilité a été déprimée par un bref séjour dans une enceinte sous pression réduite,

. diminution de la durée du sommeil barbiturique,

. présence de mouvements stéréotypés chez la souris et le rat,

. potentialisation des stéréotypies induites par l'amphétamine et l'apomorphine chez le rat;

- montrant une stimulation α-adrénergique périphérique:

. mydriase,

. exophtalmie,

. antagonisme du ptôsis réserpinique,

. antagonisme des tremblements induits par l'oxotrémorine.

III. ETUDE IMMUNOLOGIQUE

Le CRL 41 237 présente une forte activité selon le test précité des cellules formant des plages de lyse à la dose de 100 mg/kg per os, et, une activité plus faible sur la réaction d'hypersensibilité retardée aux globules rouges de mouton précitée aux doses de 0,1 mg/kg à 10 mg/kg per os.

IV. ETUDE CLINIQUE

Chez l'homme on a obtenu par voie orale de bons résultats dans le traitement des dépressions à la dose quotidienne de 10 mg de CRL 41 237.

K. ESSAIS RELATIFS AU CRL 41 241 (PRODUIT DE L'EXEMPLE 11)

Les essais neuropsychopharmacologiques entrepris selon les modalités opératoires décrites ci-dessus pour le CRL 41 153, montrent que le CRL 41 241 présente des effets

- antidépresseurs:

. antagonisme des hypothermies induites par l'apomorphine, la réserpine et l'oxotrémorine,

. diminution de la durée d'immobilité de "désespoir";

- stimulants:

. excitation avec hyper-réactivité chez la souris et le rat,

. augmentation de l'activité motrice spontanée chez la souris, reprise de l'activité motrice chez la souris habituée à son enceinte, amélioration de la récupération motrice chez la souris dont la motilité a été déprimée par un bref séjour dans une enceinte sous pression réduite,

. augmentation modérée du nombre de passages punis au test des 4 plaques chez la souris (aux doses élevées),

. diminution de la durée du sommeil barbiturique,

. présence de mouvements stéréotypés chez la souris et le rat,

. potentialisation des stéréotypies induites par l'amphétamine et l'apomorphine chez le rat.

Par ailleurs, le CRL 41 241 montre, à fortes doses:

- une composante de stimulation α-adrénergique objectivée par une diminution du ptôsis réserpinique, une mydriase modérée chez la souris et le rat,
- un antagonisme total de l'effet convulsivant de l'électrochoc,
- une diminution très nette de l'intensité des tremblements dus à l'oxotrémorine.

Le CRL 41 241 se présente donc comme une substance douée d'un éffet de type antidépresseur avec une importante composante stimulante.

L. ESSAIS RELATIFS AU CRL 41 243 (PRODUIT DE L'EXEMPLE 12)

L'étude neuropsychopharmacologique montre que le CRL 41 243 présente des effets antidépresseurs modestes, des effets sédatifs modérés (à la différence de l'ensemble des composés selon l'invention), et que, de façon paradoxale le CRL 41 243 diminue à forte dose la durée du sommeil barbiturique.

M. ESSAIS RELATIFS AU CRL 41 246 (PRODUIT DE L'EXEMPLE 13)

Les essais neuropsychopharmacologiques entrepris selon les modalités opératoires décrites ci-dessus pour le CRL 41 153, montrent que le CRL 41 246 présente des effets
- antidépresseurs:
. antagonisme des hypothermies induites par l'apomorphine, la réserpine et l'oxotrémorine,
. diminution de la durée d'immobilité de "désespoir";
- stimulants:
. excitation avec hyper-réactivité chez la souris et le rat,
. augmentation de l'activité motrice spontanée chez la souris, reprise de l'activité motrice chez la souris habituée à son enceinte, amélioration de la récupération motrice chez la souris dont la motilité a été déprimée par un bref séjour dans une enceinte sous pression réduite,
. diminution de la durée du sommeil barbiturique,
. présence de mouvements stéréotypés chez la souris et le rat,
. potentialisation des stéréotypies induites par l'amphétamine et l'apomorphine chez le rat;
- stimulants α-adrénergiques:
. mydriase,
. salivation,
. antagonisme du ptôsis réserpinique,
. antagonisme des tremblements induits par l'oxotrémorine.

**Revendications** pour les états contractants: BE, CH, DE, FR, GB, IT, LI, LU, NL, SE.

1. Dérivé de 1-(aminophényl)-2-amino-propanone caractérisé en ce qu'il est choisi parmi l'ensemble constitué par

(a) les composés répondant à la formule générale

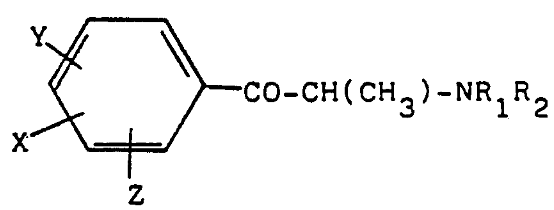

(I)

dans laquelle
X est $NH_2$,
Y est un atome d'hydrogène ou d'halogène,
Z est un atome d'hydrogène ou d'halogène,
$R_1$ est un groupe alkyle en $C_1$-$C_4$ ou un groupe cycloalkyle en $C_3$-$C_6$,
$R_2$ est l'atome d'hydrogène ou un groupe alkyle en $C_1$-$C_4$,
$R_1$ et $R_2$, considérés ensemble, peuvent former avec l'atome auquel ils sont liés un groupe N-hétérocyclique de 5 à 7 sommets, pouvant contenir un second hétéroatome choisi parmi N,O et S pouvant être substitué, ledit groupe $NR_1R_2$ étant choisi parmi l'ensemble comprenant les groupes pyrrolidino, morpholino, thiomorpholino, pipéridino, héxaméthylèneimino, pipérazino, 4-méthyl-pipérazino, 4-)β-hydro-xyéthyl)-pipérazino, 4-phénylpipérazino, 4-(p-chlorophényl)-pipérazino; et
(b) leurs sels d'addition

2. Dérivé selon la revendication 1 caractérisé en ce que dans la formule I le groupe $XYZC_6H_2$ est choisi parmi l'ensemble constitué par les groupes 4-aminophényle, 4-amino-3-chlorophényle, 4-amino-3,5-dichlorophényle, 3-aminophényle et 3-amino-4-chlorophényle.

3. 1-(4-Amino-3,5-dichlorophényl)-2-isopropylamino-propanone et ses sels d'addition.

4. 1-(4-Aminophényl)-2-morpholino-propanone et ses sels d'addition.
5. 1-(4-Aminophényl)-2-tertiobutylamino-propanone et ses sels d'addition.
6. 1-(4-Amino-3-chlorophényl)-2-isopropylamino-propanone et ses sels d'addition.
7. 1-(4-Aminophényl)-2-cyclopropylamino-propanone et sels d'addition.
8. 1-(3-Aminophényl)-2-pipéridino-propanone et ses sels d'addition.
9. Composition thérapeutique caractérisée en ce qu'elle renferme, en association avec un excipient physiologiquement acceptable, au moins un dérivé de 1-(aminophényl)-2-amino-propanone selon la revendication 1 ou l'un de ses sels d'addition non toxiques.
10. Procédé de préparation d'un dérivé de 1-(aminophényl)-2-amino-propanone de formule I selon la revendication 1, caractérisé en ce que l'on soumet un dérivé de 1-(acétylaminophényl)-2-amino-propanone de formule

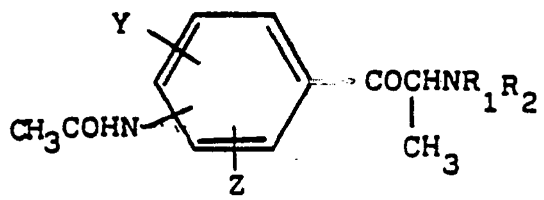

(II)

où Y, Z, $R_1$ et $R_2$ sont définis comme indiqué ci-dessus, à une réaction de désacétylation au moyen de HCl en solution aqueuse pendant au moins 0,25 h (de préférence pendant 0,25 h à 1 h) à la température de reflux du milieu réactionnel, à raison de 1 mole de II pour 0,7 à 2 1 d'acide HCl 4N.
11. Utilisation d'une substance appartenant à la famille des dérivés de 1-(aminophényl)-2-amino-propanone pour l'obtention d'un médicament antidépresseur du système nerveux central destiné à une utilisation thérapeutique vis à vis des états dépressifs, ladite utilisation étant caractérisée en ce que ladite substance est choisie parmi l'ensemble constitué par
(a) les composés répondant à la formule générale

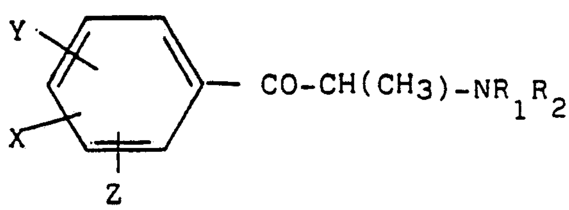

(I)

dans laquelle
X est $NH_2$,
Y est un atome d'hydrogène ou d'halogène,
Z est un atome d'hydrogène ou d'halogène,
$R_1$ est un groupe alkyle en $C_1$-$C_4$ ou un groupe cycloalkyle en $C_3$-$C_6$,
$R_2$ est l'atome d'hydrogène ou un groupe alkyle en $C_1$-$C_4$,
$R_1$ et $R_2$, considérés ensemble, peuvent former avec l'atome auquel ils sont liés un groupe N-hétérocyclique de 5 à 7 sommets, pouvant contenir un second hétéroatome choisi parmi N,O et S pouvant être substitué, ledit groupe $NR_1R_2$ étant choisi parmi l'ensemble comprenant les groupes pyrrolidino, morpholino, thiomorpholino, pipéridino, hexaméthylèneimino, pipérazino, 4-méthyl-pipérazino, 4-(β-hydroxyéthyl)-piperazino, 4-phénylpipérazino, 4-(p-chloro-phényl)-pipérazino; et,
(b) leurs sels d'addition non toxiques.
12. Utilisation d'une substance appartenant à la famille des dérivés de 1-(aminophényl)-2-amino-propanone pour l'obtention d'un médicament vasodilatateur destiné à une utilisation thérapeutique où une vasodilatation est requise, ladite utilisation étant caracterisée en ce que ladite substance est choisie parmi l'ensemble constitué par
(a) les
1-(4-amino-3-chlorophényl)-2-isopropylamino-propanone,
1-(4-aminophényl)-2-pyrrolidino-propanone,
1-(4-aminophényl)-2-éthylamino-propanone.
1-(4-aminophényl)-2-diméthylamino-propanone,
1-(4-aminophényl)-2-cyclopropylamino-propanone, et
(b) leurs sels d'addition non toxiques.
13. Utilisation d'une substance appartenant à la famille des dérivés de 1-(aminophényl)-2-amino-propanone pour l'obtention d'une médicament immunostimulant destiné à une utilisation thérapeutique où une immuno-stimulation est requise, ladite utilisation étant caractérisée en ce que ladite substance est choisie parmi

l'ensemble constitué par
(a) les
. 1-(4-amino-3,5-dichlorophényl)-2-isopropylamino-propanone,
. 1-(4-amino-3-chlorophényl)-2-isopropylamino-propanone,
. 1-(4-aminophényl)-2-morpholino-propanone,
. 1-(4-aminophényl)-2-thiomorpholino-propanone, et
(b) leurs sels d'addition non toxiques.

**Revendications** pour l'état contractant AT.

1. Procédé de préparation d'un nouveau dérivé de 1-(aminophényl)-2-amino-propanone répondant à la formule générale

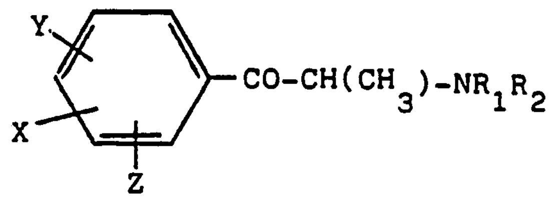

(I)

dans laquelle
X est $NH_2$,
Y est un atome d'hydrogène ou d'halogène,
Z est un atome d'hydrogène ou d'halogène,
$R_1$ est un groupe alkyle en $C_1$-$C_4$ ou un groupe cycloalkyle en $C_3$-$C_6$,
$R_2$ est l'atome d'hydrogène ou un groupe alkyle en $C_1$-$C_4$,
$R_1$ et $R_2$, considérés ensemble, peuvent former avec l'atome auquel ils sont liés un groupe N-hétérocyclique de 5 à 7 sommets, pouvant comporter un second hétéroatome, choisi parmi N,O et S et pouvant être substitué, ledit groupe hétérocyclique $NR_1$, $R_2$ étant choisi parmi l'ensemble comprenant les groupes pyrrolidino, morpholino, thiomorpholino, pipéridino, hexaméthylèneimino, pipérazino, 4-méthyl-pipérazino, 4-(β-hydroxyéthyl)-pipérazino, 4-phénylpipérazino, 4-(p-chlorophényl)-pipérazino; et ses sels d'addition;
ledit procédé étant caractérisé en ce que l'on soumet un dérivé de 1-(acétylaminophényl)-2-amino-propanone de formule

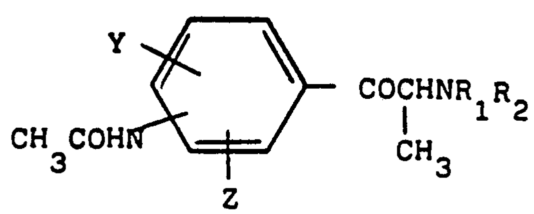

(II)

où Y, Z, $R_1$ et $R_2$ sont définis comme indiqué ci-dessus, à une réaction de désacétylation au moyen de HCl en solution aqueuse pendant au moins 0,25 h, à la température de reflux du milieu réactionnel, à raison de 1 mole de II pour 0,7 à 2 l d'acide HCl 4N.

2. Procédé selon la revendication 1, caractérisé en ce que la réaction de désacétylation est réalisée pendant 0,25 h à 1h.

**Patentansprüche** für die Vertragsstaaten BE, CH, DE, FR, GB, IT, LI, LU, NL, SE

1-(Aminophenyl)-2-aminopropanonderivat, ausgewählt aus der aus
a) Verbindungen der allgemeinen Formel

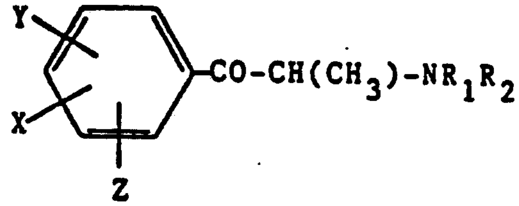

(I)

worin X $NH_2$ ist, Y H oder ein Halogenatom ist, Z H oder ein Halogenatom ist, $R_1$ $C_1$-$C_4$-Alkyl oder $C_3$-$C_6$-Cycloalkyl ist und $R_2$ H oder $C_1$-$C_4$-Alkyl ist, oder $R_1$ und $R_2$ zusammengenommen mit dem Stickstoffatomen, an welches sie gebunden sind, eine N-heterozyklische Gruppe mit 5 bis 7 Ringgleider bilden können und welche substituiert sein können, wobei die $NR_1R_2$-Gruppen aus der aus den Pyrrolidino-, Morpholino-, Thiomorpholino-, Piperidino-, Hexamethylenimino-, Piperazino-, 4-Methylpiperazino- und 4-(β-Hydroxyethyl)piperazino-, 4-Phenylpiperazino- und 4-(p-Chlorphenyl)piperazinogruppen bestehenden Gruppe ausgewählt sind, und

b) Additionssalzen hiervon bestehenden Gruppe.

2. Derivat nach Anspruch 1, worin die $XYZC_6H_2$-Gruppe in Formel I aus der aus 4-Aminophenyl, 4-Amino-3-chlorphenyl, 4-Amino-3,5-dichlorphenyl, 3- Aminophenyl und 3-Amino-4-chlorphenyl gebildeten Gruppe ausgewählt ist.

3. 1-(4-Amino-3,5-dichlorphenyl)-2-isopropylaminopropanon und seine Additionssalze.

4. 1-(4-Aminophenyl)-2-morpholinopropanon ind seine Additionssalze.

5. 1-(4-Aminophenyl)-2-tert.-butylaminopropanon und seine Additionssalze.

6. 1-(4-Amino-3-chlorphenyl)-2-isopropylaminopropanon und seine Additionssalze.

7. 1-(4-Aminophenyl)-2-cyclopropylaminopropanon und seine Additonssalze.

8. 1-(3-Aminophenyl)-2-piperidinopropanon und seine Additionssalze.

9. Therapeutische Zusammensetzung, welche in Verbindung mit einem physiologisch annehmbaren Exzipiens mindestens ein 1-(Aminophenyl)-2-aminopropanonderivat nach Anspruch 1 oder dessen nichttoxische Additionssalze enthält.

10. Verfahren zur Herstellung eines 1-(Aminophenyl)-2-amino-propanonderivates der Formel I nach Anspruch 1, wobei dieses Verfahren das Umsetzen eines 1-(Acetylaminophenyl)-2-amino-propanonderivates der Formel

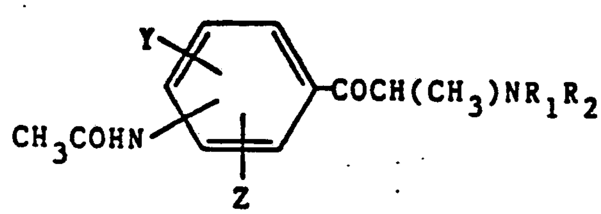

(II)

worin Y, Z, $R_1$ und $R_2$ die obenangegebenen Bedeutungen haben, in einer Deacetylierungsreaktion mit einer wäßrigen HCl-Lösung während mindestens 0,25 Stunden bei der Rückflußtemperatur des Reaktionsmediums, wobei 1 Mol Verbindung II auf 0,7 bis 2 Liter 4 N HCl verwendet wird, umfaßt.

11. Verwendung einer Substanz der Familie der 1-(Aminophenyl)-2-aminopropanonderivate zur Herstellung eines ZNS-antidepressiven Medikaments, bestimmt zur therapeutischen Anwendung bei depressiven Zuständen, wobei die Verwendung dadurch gekennzeichnet ist, daß die Substanz aus der aus

a) Verbindungen der allgemeinen Formel

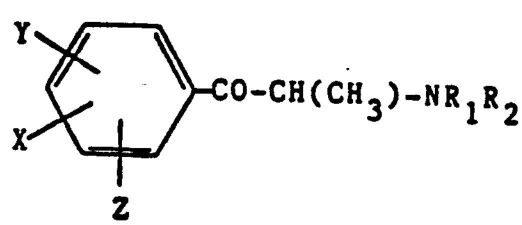

(I)

worin X $NH_2$ ist, Y H oder ein Halogenatom ist, Z H oder ein Halogenatom ist, $R_1$ und $R_2$ zusammengenommen mit dem Stickstoffatom, an welchem sie hängen, eine N-heterozyklische Gruppe mit 5 bis 7 Ringgliedern bilden können und welche substituiert sein können, wobei die $NR_1R_2$-Gruppen aus der aus den Pyrrolidino-, Morpholino-, Thiomorpholino-, Piperidino-, Hexamethylenimino-, Piperazino-, 4-Methylpiperazino-, 4-(β-Hydroxyethyl)piperazino-, 4-Phenylpiperazino- und 4-(p-Chlorphenyl)piperazinogruppen bestehenden Gruppe ausgewählt sind, und

b) nichttoxischen Salzen hiervon ausgewählt ist.

12. Verwendung einer Substanz aus der Familie der 1-(Aminophenyl)-2-aminopropanonderivate zur Herstellung eines vasodilatierenden Medikaments zur therapeutischen Anwendung, wobei eine vasodilatierende Wirkung erforderlich ist, wobei die Verwendung dadurch gekennzeichnet ist, daß die Substanz aus der aus
- 1-(4-Amino-3-chlorphenyl)-2-isopropylamino-propanon,
- 1-(4-Aminophenyl)-2-pyrrolidino-propanon,
- 1-(4-Aminophenyl)-2-ethylamino-propanon,
- 1-(4-Aminophenyl)-2-dimethylamino-propanon,
- 1-(4-Aminophenyl)-2-cyclopropylamino-propanon, und
- nichttoxischen Salzen hiervon

gebildeten Gruppe ausgewählt ist.

13. Verwendung einer Substanz aus der Familie der 1-(Aminophenyl)-2-aminopropanonderivate zur Herstellung eines immunostimulierenden Medikaments zur therapeutischen Anwendung, wobei eine immunostimulierende Wirkung erforderlich ist, wobei die Verwendung dadurch gekennzeichnet ist, daß die substanz aus der aus
- 1-(4-Amino-3,5-dichlorphenyl)-2-isopropylamino-propanon,
- 1-(4-Amino-3-chlorphenyl-2-isopropylamino-propanon,
- 1-(4-Aminophenyl)-2-morphlino-propanol,
- 1-(4-Aminophenyl)-2-thiomorpholino-propanon, und
- nichttoxischen Additionssalzen hiervon bestehenden Gruppe ausgewählt ist.

**Patentansprüche** für den Vertragsstaat AT

1. Verfahren zur Herstellung eines 1-(Aminophenyl)-2-amino-propanonderivates der allgemeinen Formel

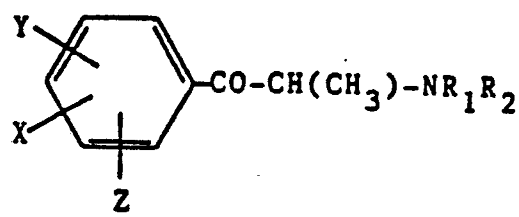

(I)

worin X $NH_2$ ist, Y H oder ein Halogenatom ist, Z H oder ein Halogenatom ist, $R_1$ $C_1$-$C_4$-Alkyl oder $C_3$-$C_6$-Cycloalkyl ist und $R_2$H oder $C_1$-$C_4$-Alkyl ist, oder $R_1$ und $R_2$ zusammengenommen mit dem Stickstoffatom, an welches sie gebunden sind, eine N-heterocyclische Gruppe mit 5 bis 7 Ringgliedern bilden können und welche substituiert sein können, wobei die $NR_1R_2$-Gruppen aus der aus den Pyrrolidino, Morpholino, Thiomorpholino, Piperidino, Hexamethylenimino, Piperazino, 4-Methylpiperazino, 4-(β-Hydroxyethyl)piperazino, 4-Phenylpiperazino- und 4-(p-Chlorphenyl)piperazino gebildeten Gruppe bestehenden Gruppe ausgewählt sind, und ihrer Additionssalze, wobei ddas Verfahren das Umsetzen einer 1-(Acetylaminophenyl)-2-amino-propanonderivates der Formel

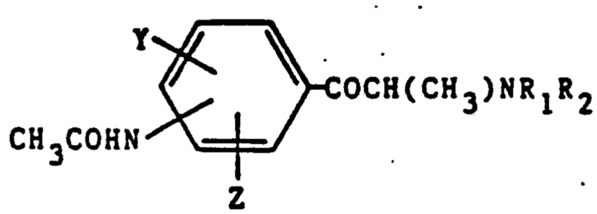

(II)

worin Y, Z, $R_1$ und $R_2$ die obenangegebenen Bedeutungen haben, in einer Deacetylierungsreaktion mit einer wäßrigen HCl-Lösung während mindestens 0,25 Stunden bei der Rückflußtemperatur des Reaktionsmediums, wobei 1 Mol Verbindung II auf 0,7 bis 2 Liter 4 N HCl verwendet wird, umfaßt.

2. Verfahren nach Anspruch 1, wobei die Deacetylierungsreaktion 0,25 bis 1 Stunde lang durchgeführt wird.

**Claims** for the contracting states: BE, CH, DE, FR, GB, IT, LI, LU, NL, SE

1. A 1-(aminophenyl)-2-aminopropanone derivative selected from the group consisting of:
a) compounds of the general formula:

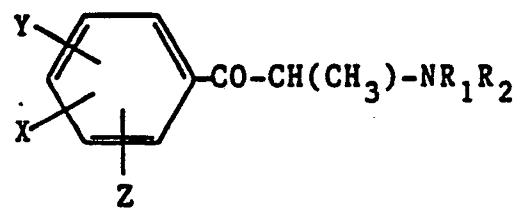

(I)

in which X is $NH_2$, Y is H or a halogen atom, Z is H or a halogen atom, $R_1$ is $C_1$-$C_4$ alkyl or $C_3$-$C_6$ cycloalkyl and $R_2$ is H or $C_1$-$C_4$ alkyl or $R_1$ and $R_2$, taken together, can form, with the nitrogen atom to which they are bonded, an N-heterocyclic group having from 5 to 7 ring members and which can be substituted, said $NR_1R_2$ groups being selected from the group comprising the pyrrolidino, morpholino, thiomorpholino, piperidino, hexamethyleneimino, piperazino, 4-methylpiperazino and 4-(β-hydroxyethyl)piperazino, 4-phenylpiperazino and 4-(p-chlorophenyl) piperazino groups; and
b) addition salts thereof.

2. A derivative according to claim 1, wherein the group $XYZC_6H_2$ in the formula I is selected from the group consisting of 4-aminophenyl, 4-amino-3-chlorophenyl, 4-amino-3,5-dichlorophenyl, 3-aminophenyl and 3-amino-4-chlorophenyl.

3. 1-(4-Amino-3,5-dichlorophenyl)-2-isopropylaminopropanone and its addition salts.

4. 1-(4-Aminophenyl)-2-morpholinopropanone and its addition salts.

5. 1-(4-Aminophenyl)-2-tert.-butylaminopropanone and its addition salts.

6. 1-(4-Amino-3-chlorophenyl)-2-isopropylaminopropanone and its addition salts.

7. 1-(4-Aminophenyl)-2-cyclopropylaminopropanone and its addition salts.

8. 1-(3-Aminophenyl)-2-piperidinopropanone and its addition salts.

9. A therapeutic composition comprising, in association with a physiologically acceptable excipient, at least one 1-(aminophenyl)-2-aminopropanone derivative according to claim 1 or one of its non- toxic addition salts.

10. A method for the preparation of a 1-(aminophenyl)-2-aminopropanone derivative of the formula I according to claim 1, said method comprising subjecting a 1-(acetylamophenyl)-2-aminopropanone derivative of the formula:

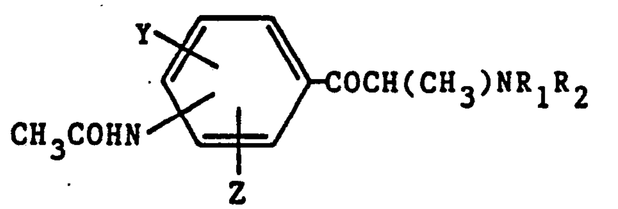

(II)

in which Y, Z, $R_1$ and $R_2$ are defined as indicated above, to a deacetylation reaction with an aqueous solution of HCl, for at least 0,25 hour, at the reflux temperature of the reaction medium, whereby 1 mol of compound II is used for 0,7 to 2 liters of 4 n HCl.

11. A use of a substance of the family of 1-(aminophenyl)-2-aminopropanone derivatives for obtaining a CNS-antidepressant medicament destined for a therapeutical use vis-à-vis depressive states, said use being characterised in that said substance is selected from the group consisting of:
a) compounds of the general formula:

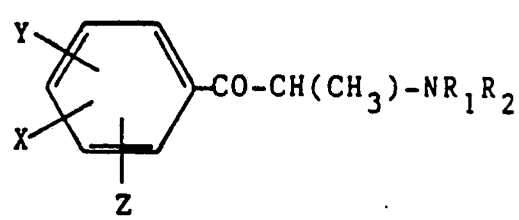

(I)

in which X is $NH_2$, is H or a halogen atom, Z is H or a halogen atom, $R_1$ is $C_1$-$C_4$ alkyl or $C_3$-$C_6$ cycloalkyl and $R_2$ is H or $C_1$-$C_4$ alkyl or $R_1$ and $R_2$, taken together, can form, with the nitrogen atom to which they are bonded, an N-heterocyclic group having from 5 to 7 ring members and which can be substituted, said $NR_1R_2$ groups being

selected from the group comprising the pyrrolidino, morpholino, thiomorpholino, piperidino, hexamethyleneimino, piperazino, 4-methylpiperazino and 4-(β-hydroxyethyl)-piperazino, 4-phenylpiperazino and 4-(p-chlorophenyl)piperazino groups; and

b) non-toxic addition salts thereof.

12. A use of a substance of the family of 1-(aminophenyl)-2-aminopropanone derivatives for obtaining a vasodilating medicament destined for a therapeutical use wherein a vasodilating action is required, said use being characterised in that said substance is selected from the group consisting of:

. 1-(4-amino-3-chlorophenyl)-2-isopropylamino-propanone,
. 1-(4-aminophenyl)-2-pyrrolidino-propanone,
. 1-(4-aminophenyl)-2-ethylamino-propanone,
. 1-(4-aminophenyl)-2-dimethylamino-propanone,
. 1-(4-aminophenyl)-2-cyclopropylamino-propanone, and
. non toxic addition salts thereof.

13. A use of a substance of the family of 1-(aminophenyl)-2-aminopropanone derivatives for obtaining an immunostimulating medicament destined for a therapeutical use wherein an immuno-stimulating action is required, said use being characterized in that said substance is selected from the group consisting of:

. 1-(4-amino-3,5-dichlorophenyl)-2-isopropylamino-propanone,
. 1-(4-amino-3-chlorophenyl)-2-isopropylamino-propanone,
. 1-(4-aminophenyl)-2-morpholino-propanone,
. 1-(4-aminophenyl)-2-thiomorpholino-propanone, and
. non-toxic addition salts thereof.

**Claims** for the contracting state: AT

1. A method for the preparation of a 1-(aminophenyl)-2-aminopropanone derivative of the general formula:

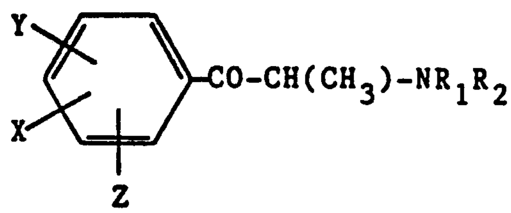

(I)

in which X is $NH_2$, is H or a halogen atom, Z is H or a halogen atom, $R_1$ is $C_1$-$C_4$ alkyl or $C_3$-$C_6$ cycloalkyl and $R_2$ is H or $C_1$-$C_4$ alkyl, or $R_1$ and $R_2$, taken together, can form, with the nitrogen atom to which they are bonded, an N-heterocyclic group having from 5 to 7 ring members and which can be substituted, said $NR_1R_2$ groups being selected from the group consisting of the pyrrolidino, morpholino, thiomorpholino, piperidino, hexamethyleneimino, piperazino, 4-methylpiperazino and 4-(β-hydroxyethyl)-piperazino, 4-phenylpiperazino and 4-(p-chlorophenyl) piperazino groups, and addition salts thereof, the said method comprising subjecting a 1-(acetylaminophenyl)-2-amino-propanone derivative of the formula:

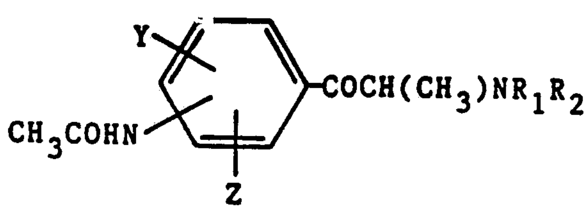

(II)

in which Y, Z, $R_1$ and $R_2$ are defined as indicated above, to a deacetylation reaction with an aqueous solution of HCl, for at least 0,25 hour, at the reflux temperature of the reaction medium, whereby 1 mol of compound II is used for 0,7 to 2 liters of 4 n HCl.

2. The method according to claim 1, wherein the deacetylation reaction is carried out for 0.25 hour to 1 hour.